# EUROPEAN PATENT APPLICATION

(11) **EP 4 491 675 A1**
(43) Date of publication of application: **15.01.2025**
(21) Application number: 24186805.8
(22) Date of filing: 05.07.2024
(51) Int. Cl.: C09B 23/04, G01N 15/14

(54) **METHOD FOR ANALYZING BLOOD CELLS, REAGENT FOR BLOOD CELL ANALYSIS, REAGENT KIT FOR BLOOD CELL ANALYSIS, AND METHOD FOR FLUORESCENTLY STAINING BLOOD CELLS**

(30) Priority: 10.07.2023 JP 2023113305; 10.07.2023 JP 2023113309; 10.07.2023 JP 2023113311
(71) Applicant: SYSMEX CORPORATION, Kobe-shi Hyogo 651-0073 (JP)
(72) Inventor: MATSUBA, Hiroaki, Kobe-shi, Hyogo 651-0073 (JP)
(74) Representative: Hoffmann Eitle

(57) **Abstract**

Disclosed is a method for analyzing blood cells, the method includes:
irradiating a measurement sample that contains particles stained with a fluorescent dye with at least one light, to acquire fluorescence information and scattered light information generated from the particle; and
classifying blood cells from the particles in the measurement sample, with reference to the fluorescence information and the scattered light information,
the fluorescent dye being represented by formula (I) below: 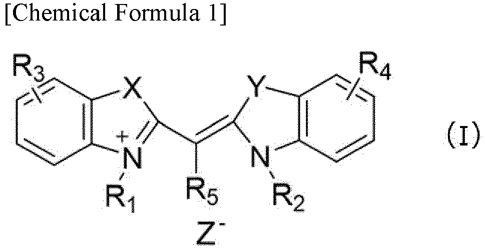 (where, each of R₁ and R₂, identically or differently, is a halogen, an alkyl group having 1 to 18 carbon atoms, an alkenyl group having 3 to 20 carbon atoms, an alkyl group having 1 to 18 carbon atoms and a hydroxy group, an alkyl group having 1 to 18 carbon atoms and a carboxy group, an alkyl group having 1 to 18 carbon atoms and a sulfo group, -(CH₂)ₘ-NR₆R₇, -(CH₂)ₙ-O-R₈, or a benzyl group optionally having a substituent;
each of R₃ and R₄, identically or differently, is a hydrogen atom, a halogen, an alkyl group having 1 to 18 carbon atoms, a phenyl group optionally having a substituent, an alkoxy group having 1 to 6 carbon atoms, or -(CH₂)ₘ-O-R₈;
R₅ is a hydrogen atom, a halogen, an alkyl group having 1 to 6 carbon atoms, an alkyl group having 1 to 6 carbon atoms and a hydroxy group, an alkyl group having 1 to 6 carbon atoms and a carboxy group, an alkyl group having 1 to 6 carbon atoms and a sulfo group, a phenyl group optionally having a substituent, a benzyl group optionally having a substituent, or -(CH₂)ₙ-O-R₆;
each of R₆ and R₇, identically or differently, is a hydrogen atom or an alkyl group having 1 to 18 carbon atoms;
R₈ is a hydrogen atom, an alkyl group having 1 to 18 carbon atoms, or a phenyl group optionally having a substituent;
m is an integer of 1 to 18, and n is an integer of 1 to 6;
each of X and Y, identically or differently, is a sulfur atom, an oxygen atom, a selenium atom, or CR₉R₁₀;
each of R₉ and R₁₀, identically or differently, is an alkyl group having 1 to 3 carbon atoms; and
Z⁻ is a counter ion).

## Description

### FIELD OF THE INVENTION

The present invention relates to a method for analyzing blood cells. The present invention also relates to a reagent for blood cell analysis. The present invention also relates to a reagent kit for blood cell analysis. The present invention also relates to a method for fluorescently staining blood cells.

### BACKGROUND

Blood includes various blood cell components such as red blood cell, white blood cell, and platelet. Analysis of these blood cells can provide information valuable for clinical diagnosis of various diseases. A method with use of an automated hematology analyzer has been known for use in blood cell analysis. The analysis with use of the automated hematology analyzer usually measures a sample, which is prepared with use of a dedicated reagent suited to types of the blood cells. A reagent commonly applicable to a plurality of types of blood cells has, however, been desired from the viewpoints of labor-saving of inspection and simplicity of the apparatus. For example, WO2022/100717 describes that white blood cell was classified and counted with use of one kind of fluorescent dye and a hemolysis reagent, and that reticulocyte and platelet are classified and counted with use of the same fluorescent dye and a dilution reagent.

### SUMMARY OF THE INVENTION

A recent known method of analysis is such as irradiating blood cells with a plurality of lights of different wavelengths, with use of a hematology analyzer equipped with a plurality of light sources. There is, therefore, a need for a method for analyzing blood cells, with use of a reagent which is applicable to blood cell analysis under such plurality of lights. It is therefore an object of the present invention to provide a means for blood cell analysis, with use of a reagent which is commonly applicable to classification of white blood cell and classification of reticulocyte and platelet, as well as applicable to blood cell analysis under a plurality of lights.

The present inventor found that a cyanine-based fluorescent dye that can be excited by light, whose wavelength is 315 nm or longer and 600 nm or shorter, and can bind to a nucleic acid, is commonly applicable to classification of white blood cell, classification of reticulocyte and platelet, as well as blood cell analysis under a plurality of lights, thereby completing the invention described in [1] to [19] below.
[1] A method for analyzing blood cells, the method including:
   irradiating a measurement sample that contains particles stained with a fluorescent dye with at least one light, to acquire fluorescence information and scattered light information generated from the particle; and
   classifying blood cells from the particles in the measurement sample, with reference to the fluorescence information and the scattered light information,
   the fluorescent dye being represented by formula (I) below: 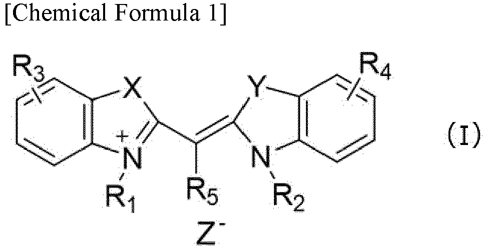
      (where, each of R₁ and R₂, identically or differently, is a halogen, an alkyl group having 1 to 18 carbon atoms, an alkenyl group having 3 to 20 carbon atoms, an alkyl group having 1 to 18 carbon atoms and a hydroxy group, an alkyl group having 1 to 18 carbon atoms and a carboxy group, an alkyl group having 1 to 18 carbon atoms and a sulfo group, -(CH₂)ₘ-NR₆R₇, -(CH₂)ₙ-O-R₈, or a benzyl group optionally having a substituent;
      each of R₃ and R₄, identically or differently, is a hydrogen atom, a halogen, an alkyl group having 1 to 18 carbon atoms, a phenyl group optionally having a substituent, an alkoxy group having 1 to 6 carbon atoms, or -(CH₂)ₘ-O-R₈;
      R₅ is a hydrogen atom, a halogen, an alkyl group having 1 to 6 carbon atoms, an alkyl group having 1 to 6 carbon atoms and a hydroxy group, an alkyl group having 1 to 6 carbon atoms and a carboxy group, an alkyl group having 1 to 6 carbon atoms and a sulfo group, a phenyl group optionally having a substituent, a benzyl group optionally having a substituent, or -(CH₂)ₙ-O-R₆;
      each of R₆ and R₇, identically or differently, is a hydrogen atom or an alkyl group having 1 to 18 carbon atoms;
      R₈ is a hydrogen atom, an alkyl group having 1 to 18 carbon atoms, or a phenyl group optionally having a substituent;
      m is an integer of 1 to 18, and n is an integer of 1 to 6;
      each of X and Y, identically or differently, is a sulfur atom, an oxygen atom, a selenium atom, or CR₉R₁₀;
      each of R₉ and R₁₀, identically or differently, is an alkyl group having 1 to 3 carbon atoms; and
      Z⁻ is a counter ion).
[2] The method according to [1], in which in formula (I), with either X or Y given by an oxygen atom, the other is a sulfur atom, a selenium atom, or C(CH₃)₂.
[3] The method according to [1] or [2], in which the fluorescent dye is at least one selected from the group consisting of 3-ethyl-2-[(3-ethyl-2-benzothiazolinylidene)methyl] benzothiazolium, 3-ethyl-2-[(3-ethyl-6-methyl-2-benzothiazolinylidene)methyl]-6-methyl-benzothiazolium, 3-ethyl-2-[(3-ethyl-2-benzothiazolinylidene)methyl]benzoxazolium, 3-ethyl-6-methyl-2-[(3-ethyl-6-methyl-2-benzothiazolinylidene)methyl]benzothiazolium, 3-methyl-2-[(1,3,3-trimethyl-2-indolinylidene)methyl]benzothiazolium, and 1,3,3-trimethyl-2-[(3-methyl-2-benzoxazolinylidene)methyl]-3H-indolium.
[4] The method according to any one of [1] to [3], in which the measurement sample is prepared by mixing the fluorescent dye, a hemolysis reagent, and whole blood, and
   in the step of classifying, white blood cell in the measurement sample is classified into subpopulations.
[5] The method according to [4], in which the at least one light is a light of a first wavelength from 315 nm or longer and 600 nm or shorter,
   the fluorescence information is information related to fluorescence generated from the fluorescent dye on the particles upon irradiation of the measurement sample with the light of the first wavelength, and
   the scattered light information is information related to side-scattered light generated from the particles upon irradiation of the measurement sample with the light of the first wavelength.
[6] The method according to [4], in which the at least one light is a light of a first wavelength from 315 nm or longer and 600 nm or shorter, and a light of a second wavelength from 610 nm or longer and 750 nm or shorter,
   the fluorescence information is information related to fluorescence generated from the fluorescent dye on the particles upon irradiation of the measurement sample with the light of the first wavelength, and
   the scattered light information is information related to side-scattered light generated from the particles upon irradiation of the measurement sample with the light of the first wavelength,
   and information related to side-scattered light generated from the particles upon irradiation of the measurement sample with the light of the second wavelength.
[7] The method according to [5] or [6], in which the fluorescence information is fluorescence intensity, and the scattered light information is side-scattered light intensity, and
   in the step of classifying, white blood cell in the measurement sample is classified into subpopulations, with reference to the fluorescence intensity and the side-scattered light intensity.
[8] The method according to [7], wherein a scattergram is created with reference to the fluorescence intensity and the side-scattered light intensity, and the white blood cell is classified into subpopulations.
[9] The method according to [8], in which the subpopulations include lymphocyte population, monocyte population, and granulocyte population.
[10] The method according to [8], in which the subpopulations include lymphocyte population, monocyte population, neutrophil population, eosinophil population, and basophil population.
[11] The method according to any one of [1] to [3], in which the measurement sample is prepared by mixing the fluorescent dye, a dilution reagent, and whole blood, and
   in the step of classifying, reticulocyte and platelet are classified from the particles in the measurement sample.
[12] The method according to [11], in which the measurement sample is free of a hemolysis reagent.
[13] The method according to [11] or [12], in which the at least one light is a light of a first wavelength from 315 nm or longer and 600 nm or shorter, and a light of a second wavelength from 610 nm or longer and 750 nm or shorter,
   the fluorescence information is information related to fluorescence generated from the fluorescent dye on the particles upon irradiation of the measurement sample with the light of the first wavelength, and
   the scattered light information is information related to forward-scattered light generated from the particles upon irradiation of the measurement sample with the light of the second wavelength.
[14] The method according to [13], in which the fluorescence information is fluorescence intensity, and the scattered light information is forward-scattered light intensity, and
   in the step of classifying, the particles in the measurement sample are classified into reticulocyte, platelet, and mature red blood cell, with reference to the fluorescence intensity and the forward-scattered light intensity.
[15] The method according to [14], wherein a scattergram is created with reference to the fluorescence intensity and the forward-scattered light intensity, and the particles in the measurement sample are classified into reticulocyte population, platelet population, and mature red blood cell population, on the scattergram.
[16] The method according to any one of [1] to [15], in which the first wavelength is 315 nm or longer and 490 nm or shorter.
[17] A reagent for blood cell analysis, including the fluorescent dye represented by formula (I) above, and for use in the method according to any one of [1] to [16].
[18] A reagent kit for blood cell analysis, including a staining reagent that contains the fluorescent dye represented by formula (I) above, and a hemolysis reagent, for use in the method according to any one of [1] to [16].
[19] A method for fluorescently staining blood cells, the method including contacting a fluorescent dye with the blood cells, the fluorescent dye being represented by formula (I) below: 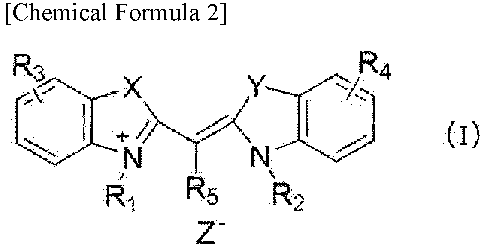
   (where, each of R₁ and R₂, identically or differently, is a halogen, an alkyl group having 1 to 18 carbon atoms, an alkenyl group having 3 to 20 carbon atoms, an alkyl group having 1 to 18 carbon atoms and a hydroxy group, an alkyl group having 1 to 18 carbon atoms and a carboxy group, an alkyl group having 1 to 18 carbon atoms and a sulfo group, -(CH₂)ₘ-NR₆R₇, -(CH₂)ₙ-O-R₈, or a benzyl group optionally having a substituent;
   each of R₃ and R₄, identically or differently, is a hydrogen atom, a halogen, an alkyl group having 1 to 18 carbon atoms, a phenyl group optionally having a substituent, an alkoxy group having 1 to 6 carbon atoms, or -(CH₂)ₘ-O-R₈;
   R₅ is a hydrogen atom, a halogen, an alkyl group having 1 to 6 carbon atoms, an alkyl group having 1 to 6 carbon atoms and a hydroxy group, an alkyl group having 1 to 6 carbon atoms and a carboxy group, an alkyl group having 1 to 6 carbon atoms and a sulfo group, a phenyl group optionally having a substituent, a benzyl group optionally having a substituent, or -(CH₂)ₙ-O-R₆;
   each of R₆ and R₇, identically or differently, is a hydrogen atom, or an alkyl group having 1 to 18 carbon atoms;
   R₈ is a hydrogen atom, an alkyl group having 1 to 18 carbon atoms, or a phenyl group optionally having a substituent;
   m is an integer of 1 to 18, and n is an integer of 1 to 6;
   each of X and Y, identically or differently, is a sulfur atom, an oxygen atom, a selenium atom, or CR₉R₁₀;
   each of R₉ and R₁₀, identically or differently, is an alkyl group having 1 to 3 carbon atoms; and
   Z⁻ is a counter ion).

The present invention can provide a method for analyzing blood cells with use of a fluorescent dye that can be used in common for the classification of white blood cell, classification of platelet and reticulocyte, as well as applicable to blood cell analysis under a plurality of lights. The present invention can also provide a reagent for blood cell analysis and a reagent kit for blood cell analysis, which are applicable to this method. The present invention can also provide a method for fluorescently staining blood cells with use of the aforementioned fluorescent dye.

### BRIEF DESCRIPTION OF THE DRAWINGS

Fig. 1 is a perspective view illustrating an analysis system suitable for the method for analyzing blood cells of this embodiment.
Fig. 2 is a drawing illustrating a fluid circuit in a measurement unit, which involves a specimen suction unit, a sample preparation unit, and a flow cytometer (FCM) detector.
Fig. 3 is a block diagram illustrating a structure of the measurement unit.
Fig. 4 is a schematic drawing illustrating an exemplary structure of an optical system of the FCM detector.
Fig. 5 is a schematic drawing illustrating various kinds of light emitted from a particle that passes through a flow cell under irradiation with light, received by the optical system of the FCM detector.
Fig. 6 is a block diagram illustrating a structure of an analysis unit.
Fig. 7 is a flowchart illustrating a flow of operations of the analysis system.
Fig. 8 is a flowchart illustrating procedures of a measurement process in a first embodiment.
Fig. 9 is a flowchart illustrating procedures of a measurement process in a second embodiment, where, "WBC" stands for white blood cell.
Fig. 10 is an absorption spectral chart of fluorescent dyes in ethanol.
Fig. 11A is a perspective view illustrating an exemplary reagent for blood cell analysis of this embodiment.
Fig. 11B is a perspective view illustrating an exemplary reagent kit for blood cell analysis of this embodiment.
Fig. 12 is a flowchart illustrating procedures of an analysis process in the first embodiment.

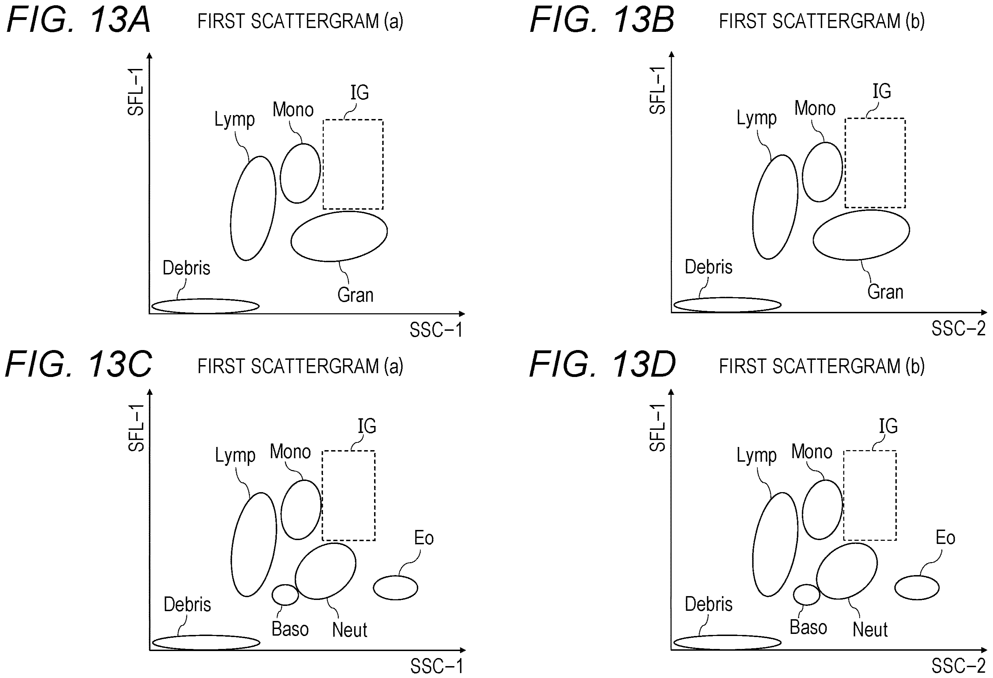

Fig. 13 contains schematic drawings, where (A) and (C) are first scattergrams (a), with a first side-scattered light (SSC-1) intensity plotted on the abscissa, and with a first fluorescence (SFL-1) intensity plotted on the ordinate, exemplifying positions where the individual subpopulations of white blood cell appear; and (B) and (D) are first scattergrams (b), with the second side-scattered light (SSC-2) intensity plotted on the abscissa, and with the SFL-1 intensity plotted on the ordinate, exemplifying positions where the individual subpopulations of white blood cell appear. The granulocyte is a generic term for neutrophil, eosinophil, and basophil.
Fig. 14 is a flowchart illustrating procedures of an analysis process in the second embodiment.

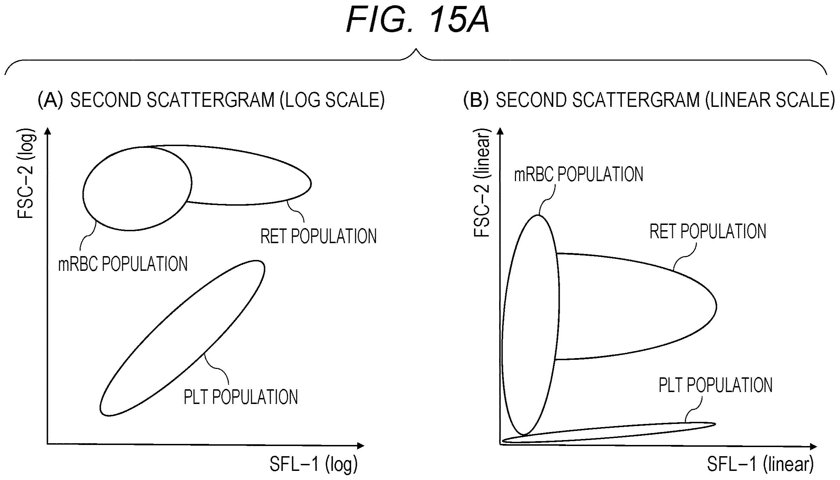

Fig. 15A contains schematic drawings, where (A) is a second scattergram (in logarithmic scale), with a logarithm of the first fluorescence intensity (SFL-1(log)) plotted on the abscissa, and with a logarithm of the second forward-scattered light intensity (FSC-2(log)) plotted on the ordinate, exemplifying positions where a RET population, an mRBC population, and a PLT population appear. (B) is a second scattergram (in linear scale), with the SFL-1 intensity plotted on the abscissa, and with the FSC-2 intensity plotted on the ordinate, exemplifying positions where the RET population, the mRBC population, and the PLT population appear.
Fig. 15B contains schematic drawings, where (A) is the second scattergram (in logarithmic scale), exemplifying areas where mRBC and RET individually appear; and (B) is the second scattergram (in linear scale), exemplifying areas where mRBC and RET individually appear.
Fig. 16 is a schematic drawing of the second scattergram (in linear scale), exemplifying an area where mRBC appears, a low fluorescence rate (LFR) area, a middle fluorescence rate (MFR) area, and a high fluorescence rate (HFR) area.
Fig. 17 contains schematic drawings, where (A) is the second scattergram (in logarithmic scale), exemplifying an area where immature platelet appears; and (B) is the second scattergram (in logarithmic scale), exemplifying an area where large platelet appears.
Fig. 18 contains a scattergram of Comparative Example 1, and the first scattergrams (a) and (b) of Example 1.
Fig. 19 contains the first scattergrams (a) and (b) of Example 1.
Fig. 20 contains scattergrams of Comparative Examples 2 and 3, and the second scattergrams of Example 2.
Fig. 21 contains the second scattergrams of Example 2.

### DETAILED DESCRIPTION OF THE PREFERRED EMBODIMENTS

### (Analysis System)

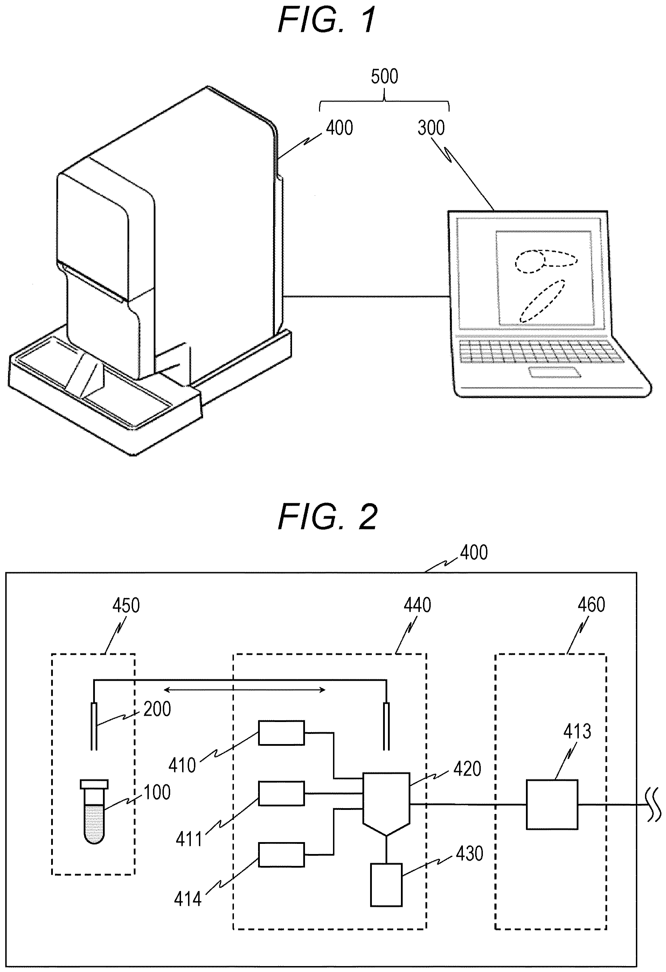

First, an analysis system suitable for a method for analyzing blood cells of this embodiment will be described, while referring to Fig. 1. An analysis system 500 has a measurement unit 400 as a measurement apparatus, and an analysis unit 300 as an analyzer. The analysis unit 300 is typically a personal computer having installed thereon software for analyzing specimen to be measured. The analysis unit 300 also takes part in operational control of the measurement unit 400. The analysis system 500 may alternatively be structured, with the analysis unit 300 incorporated in the measurement unit 400.

The measurement unit 400 is a unit for measuring the specimen, and contains a flow cytometer. In the measurement unit 400, the specimen and a reagent are mixed to prepare a measurement sample. For classification of white blood cell in the specimen, the measurement sample is prepared with use of reagents, which are a staining reagent and a hemolysis reagent. For classification of reticulocyte and platelet in the specimen, the measurement sample is prepared with use of reagents, which are a staining reagent and a dilution reagent. The staining reagent contains a fluorescent dye for staining particle. The hemolysis reagent contains a surfactant and a solvent. The dilution reagent contains a buffering agent and a solvent. The specimen, the staining reagent, the hemolysis reagent, and the dilution reagent will be detailed later. In analysis of the particles in the specimen with this analysis system, particles stained with the fluorescent dye in the measurement sample are analyzed. The "particles in the measurement sample" refers to elements contained in the measurement sample, which can be individually measured by an FCM detector 460 described later. The particles in the measurement sample are exemplified by cell contained in the specimen, red blood cell ghosts, platelet aggregation, lipid particle, fungi, and bacteria. The term "cell" encompasses various types of blood cell and platelet. The red blood cell ghost may be produced also due to hemolysis caused by other than the hemolysis reagent, that is, caused by physical impact on blood collection tube; or low pH or low osmotic pressure of the measurement sample.

The measurement sample is measured with an FCM detector 460 (see Fig. 2), described later, in the measurement unit 400. From the measurement, acquired are an optical signal related to fluorescence emitted from the fluorescent dye on the stained particles in the measurement sample, and an optical signal related to scattered light emitted from the particle. The "fluorescent dye on the particle" refers to a fluorescent dye, which is bound to the particle by staining. Mode of binding between the particle and the fluorescent dye is not particularly limited, as long as the particle and the fluorescent dye are integrally measurable by the flow cytometer. The acquired optical signals are subjected to A/D conversion. Digital data is thus acquired. The analysis unit 300 analyzes the digital data obtained by the measurement unit 400, to identify and/or classify the particles in the measurement sample.

A structure of a fluid system in the measurement unit 400 will be described, while referring to Fig. 2. The measurement unit 400 contains a sample preparation unit 440, a specimen suction unit 450, and the FCM detector 460. The sample preparation unit 440 contains a reagent containers 410, 411 and 414, a reaction chamber 420, and a waste liquid chamber 430. For example, the reagent container 410 stores the dilution reagent, the reagent container 411 stores the hemolysis reagent, and the reagent container 414 stores the staining reagent. The reagent containers 410, 411 and 414 are individually connected through liquid feeding tubes to the reaction chamber 420. In the sample preparation unit 440, the dilution reagent and the staining reagent are injected through the individual liquid feeding tubes into the reaction chamber 420. The specimen suction unit 450 contains a suction tube 200. The suction tube 200 sucks the specimen contained in the blood collection tube 100, and discharges the specimen into the reaction chamber 420. The reaction chamber 420 is connected through a liquid feeding tube, to the FCM detector 460.

The reaction chamber 420 is a container for preparing a measurement sample. In the reaction chamber 420, mixed are the specimen, the staining reagent that contains a fluorescent dye, and the hemolysis reagent or the dilution reagent, to prepare the measurement sample. The measurement sample in the reaction chamber 420 is fed through the liquid feeding tube to the FCM detector 460, and measured. The FCM detector 460 acquires various optical signals emitted from each particle in the measurement sample. After completion of the measurement with the FCM detector 460, the measurement sample that remained in the reaction chamber 420 is discarded in the waste liquid chamber 430. The reaction chamber 420 is washed with an unillustrated washing mechanism, before preparation of the next measurement sample.

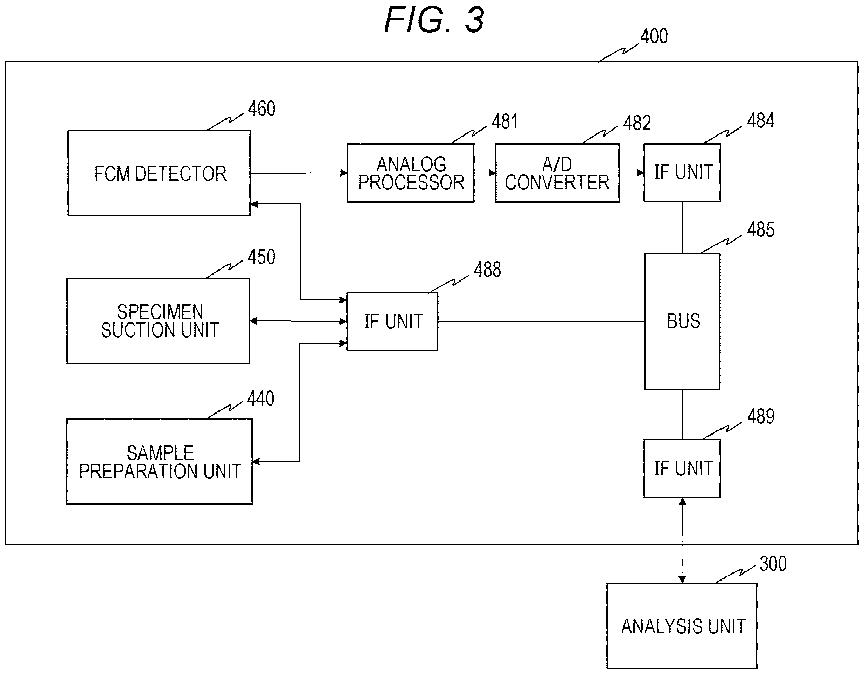

Electrical connection of the individual components in the measurement unit 400 will be described, while referring to Fig. 3. The sample preparation unit 440, the specimen suction unit 450, and the FCM detector 460 are connected to an interface (IF) unit 488. The FCM detector 460 is connected to an analog processor 481 and an A/D converter 482. Analog processor 481 processes an analog signal output from the FCM detector 460, and the A/D converter 482 converts the analog signal output from the analog processor 481 into a digital signal. The measurement unit 400 is connected through an IF unit 489 to the analysis unit 300. An IF unit 484, the IF unit 488, and the IF unit 489 are connected to a bus.

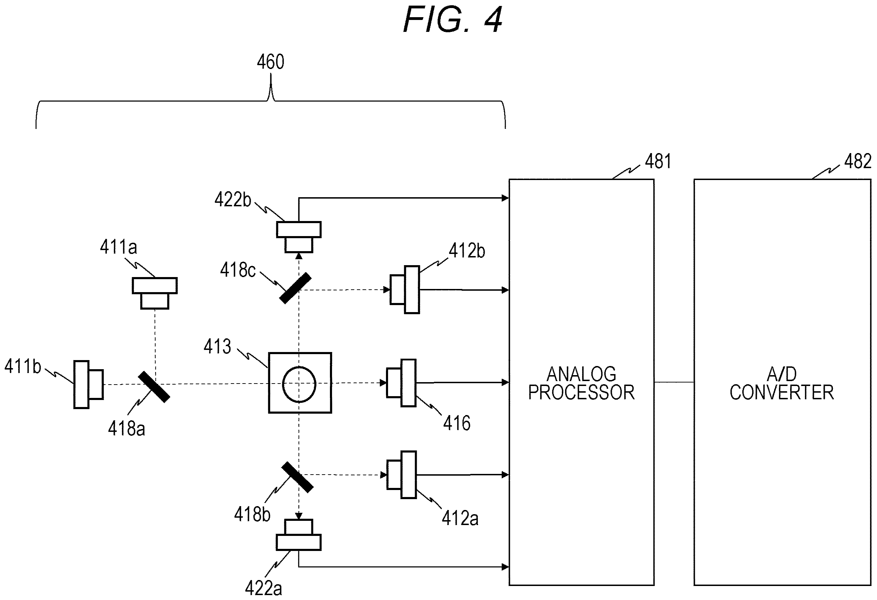

The FCM detector 460, the analog processor 481, and the A/D converter 482 will be described, while referring to Fig. 4. The FCM detector 460 has a first light source 411a, a second light source 411b, a flow cell 413, dichroic mirrors 418a, 418b, 418c, side-scattered light receivers 412a, 412b, a forward-scattered light receiver 416, and side-fluorescence receivers 422a, 422b. The first light source 411a and the second light source 411b emit light of different wavelengths. For example, the first light source 411a emits light of a first wavelength, and the second light source 411b emits light of a second wavelength. The first wavelength is preferably a wavelength that hemoglobin can absorb. Such wavelength typically falls in the range from 315 nm or longer to 600 nm or shorter, preferably 400 nm or longer and 490 nm or shorter, more preferably 400 nm or longer and 450 nm or shorter, and yet more preferably 400 nm or longer and 410 nm or shorter. The second wavelength is different from the first wavelength, whose wavelength is preferably not absorbable by hemoglobin. Such wavelength typically falls in the range from 610 nm or longer to 750 nm or shorter, preferably 620 nm or longer and 700 nm or shorter, and more preferably 633 nm or longer and 643 nm or shorter. A semiconductor laser light source, for example, is applicable to the first and second light sources.

The measurement sample prepared in the reaction chamber 420 is fed to the flow cell 413 of the FCM detector 460. In the illustration of Fig. 4, the measurement sample flows in a direction perpendicular to this page. With the measurement sample kept flowed through the flow cell 413, light emitted from the first light source 411a is reflected on the dichroic mirror 418a, and then irradiated on each particle in the measurement sample that flows through the flow cell 413. Light emitted from the second light source 411b transmits through the dichroic mirror 418a, and then irradiated on each particle in the measurement sample that flows through the flow cell 413.

In the example of Fig. 4, the forward-scattered light receiver 416 is arranged so as to receive forward-scattered light emitted from the particle upon being irradiated with the light from the second light source 411b. The light receiver 416 may alternatively be arranged so as to receive the forward-scattered light emitted from the particle upon being irradiated with the light from the first light source 411a. Still alternatively, an additional light receiver may be arranged so as to receive the forward-scattered light emitted from the particle upon being irradiated with the light from the first light source 411a. The forward-scattered light is typically a scattered light which may be received at a receiving angle of 0 to approximately 20 degrees. The forward-scattered light receiver 416 is typically a photodiode. The side-scattered light (first side-scattered light) that corresponds to the light irradiated by the first light source 411a is reflected on the dichroic mirror 418b, and then received by the side-scattered light receiver 412a. The side-scattered light (second side-scattered light) corresponded to the light irradiated by the second light source 411b is reflected on the dichroic mirror 418c, and then received by the side-scattered light receiver 412b. The side-scattered light is typically a scattered light which may be received at a receiving angle of approximately 20 degrees to approximately 90 degrees. The side-scattered light receivers 412a and 412b each are typically a photodiode.

The fluorescent dye represented by formula (I) above is excited by the first wavelength. The side-fluorescence (first side-fluorescence) that corresponds to the light emitted upon excitation of the fluorescent dye transmits through the dichroic mirror 418b, and then received by the side-fluorescence receiver 422a. Although the method for analyzing blood cells of this embodiment does not use any fluorescent dye that can be excited at the second wavelength, the example of Fig. 4 illustrates a structure capable of receiving the side-fluorescence (second side-fluorescence) that corresponds to the light emitted upon excitation of the fluorescent dye, for the convenience of explaining the optical system. That is, the second side-fluorescence transmits through dichroic mirror 418c, and then received by the side-fluorescence receiver 422b. The side-fluorescence receivers 422a and 422b each is typically an avalanche photodiode.

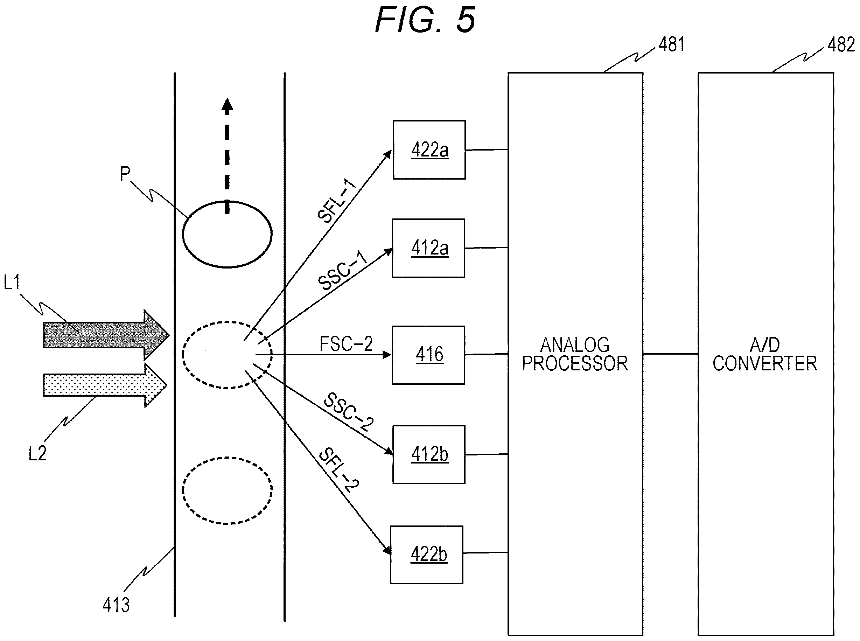

Relations of the various types of light emitted from a particle P that passes through the flow cell 413, with the optical system of the FCM detector 460 will be explained, while referring to Fig. 5. In Fig. 5, the light emitted from the first light source 411a is light L1 of the first wavelength, and the light emitted from the second light source 411b is light L2 of the second wavelength. Upon being irradiated with lights L1 and L2, the particle P that passes through the flow cell 413 emits forward-scattered lights that individually correspond to the light of the first wavelength and the light of the second wavelength, ahead of the direction of travel of light. Since the light receiver 416 herein receives the forward-scattered light that corresponds to the light of the second wavelength, Fig. 5 illustrates only the second forward-scattered light (FSC-2) that corresponds to the light of the second wavelength, while omitting the first forward-scattered light (FSC-1) that corresponds to the light of the first wavelength. Referring now to Fig. 5, there are generated the first side-scattered light (SSC-1) that corresponds to light of the first wavelength, and the second side-scattered light (SSC-2) that corresponds to the light of the second wavelength, emitted laterally with respect to the direction of travel of light. With the particle P stained with the fluorescent dye that can be excited with the light of the first wavelength, obtainable is the first side-fluorescence (SFL-1) excited with the light of the first wavelength, emitted laterally with respect to the direction of travel of light. Although the method for analyzing blood cells of this embodiment does not use any fluorescent dye that can be excited at the second wavelength, the example of Fig. 5 illustrates a state in which both of SFL-1, and the second side-fluorescence (SFL-2) excited by the light of the second wavelength are generated, laterally to the direction of travel of light, for the convenience of explaining the optical system.

As has been described, FSC-2, SSC-1, SFL-1, SSC-2, and SFL-2 are received by the light receivers 416, 412a, 422a, 412b, and 422b, respectively. Each light receiving element outputs a wavy electric signal (also referred to as an analog signal) that contains a pulse corresponded to the received light intensity. The analog signal corresponded to FSC-2 is also referred to as the "second forward-scattered light signal", the analog signal corresponded to the SSC-1 also as the "first side-scattered light signal", the analog signal corresponded to SFL-1 also as the "first fluorescence signal", the analog signal corresponded to SSC-2 also as the "second side-scattered light signal", and the analog signal corresponded to SFL-2 also as the "second fluorescence signal". In a case where FSC-1 is received, the analog signal corresponded to FSC-1 is also referred to as the "first forward-scattered light signal". One pulse of each analog signal corresponds to one particle (one cell, for example).

The analog signals corresponded to the various types of light are individually input to the analog processor 481, and subjected to processes such as noise removal and smoothing. The A/D converter 482 samples analog signals output from the analog processor 481, at a predetermined sampling rate (for example, 1024 points sampled at 10 nanosecond intervals, or 128 points sampled at 80 nanosecond intervals, or 64 points sampled at 160 nanosecond intervals). The A/D converter 482 digitizes the sampled analog signal, to produce waveform data. The A/D converter 482 samples and digitizes five types of analog signals that correspond to the individual cells that flow through the flow cell 413, to create waveform data including the second forward-scattered light signal, the first side-scattered light signal, the first fluorescence signal, the second side-scattered light signal, and the second fluorescence signal. In a case where FSC-1 is received, also waveform data of the first forward-scattered light signal is created. The A/D converter 482 also calculates a feature parameter that represents a morphological feature of each cell, from the waveform data of each signal. Such feature parameter is exemplified by peak value (height of pulse peak), pulse width, pulse area, transmittance, Stokes shift, ratio, temporal change, and values correlated thereto.

Optical information can be the aforementioned feature parameter. The optical information includes the fluorescence information and the scattered light information. The fluorescence information relates to the fluorescence generated from the fluorescent dye on the stained particle, upon irradiation of the measurement sample with light of the first wavelength. The fluorescence information is not particularly limited as long as it reflects the amount of the fluorescent dye that has stained the nucleic acid in nucleated cell. The fluorescence information is preferably peak value or pulse area of the first fluorescence signal, and is more preferably the peak value of the first fluorescence signal. The peak value of the first fluorescence signal herein is also referred to as "first fluorescence intensity". The first fluorescence intensity is understood to be intensity of fluorescence from the fluorescent dye on the particle irradiated with the light of the first wavelength.

The scattered light information acquired herein may alternatively be a plurality of types of scattered light, which are typically a first scattered light information, a second scattered light information, and a third scattered light information. For example, the first scattered light information can be information related to the side-scattered light generated from the particles, upon irradiation of the measurement sample with the light of the first wavelength. The second scattered light information can be information related to the side-scattered light generated from the particles, upon irradiation of the measurement sample with the light of the second wavelength. The third scattered light information can be information related to the forward-scattered light generated from the particles, upon irradiation of the measurement sample with the light of the second wavelength. Information on the side-scattered light is not particularly limited as long as it reflects internal information such as complexity of cellular structure, granule characteristic, nuclear structure, and degree of segmentation. Information on the forward-scattered light is not particularly limited as long as it reflects size of the particle. The first scattered light information is preferably peak value or pulse area of the first side-scattered light signal, and is more preferably the peak value of the first side-scattered light signal. The second scattered light information is preferably a peak value or a pulse area of the second side-scattered light signal, and is more preferably the peak value of the second side-scattered light signal. The third scattered light information is preferably a peak value or a pulse area of the second forward-scattered light signal, and is more preferably the peak value of the second forward-scattered light signal. In this specification, the peak value of the first side-scattered light signal is also referred to as "first side-scattered light intensity", the peak value of the second side-scattered light signal is also referred to as "second side-scattered light intensity", and the peak value of the second forward-scattered light signal is also referred to as "second forward-scattered light intensity". The first side-scattered light intensity is understood to be intensity of the first side-scattered light emitted from the particle irradiated with the light of the first wavelength. The second side-scattered light intensity is understood to be intensity of the second side-scattered light emitted from the particle irradiated with the light of the second wavelength. The second forward-scattered light intensity is understood to be intensity of the forward-scattered light emitted from the particle irradiated with the light of the second wavelength.

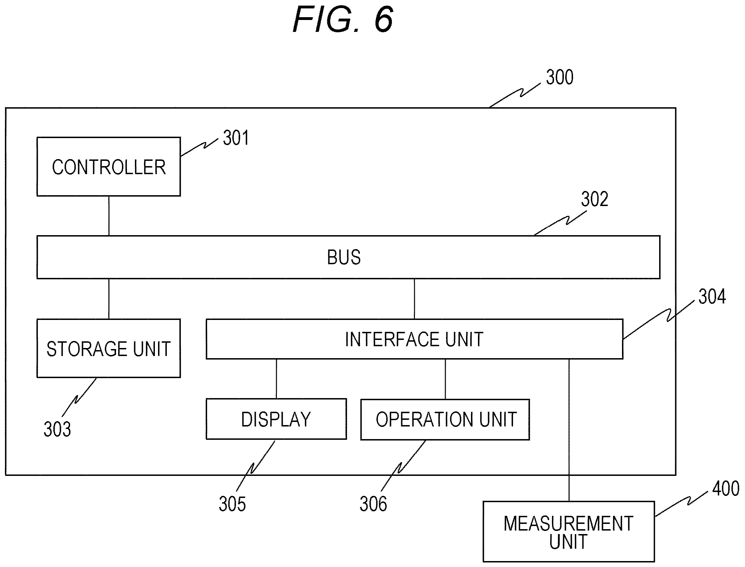

Referring now to Fig. 6, the analysis unit 300 is electrically connected through the interface unit 304 to the measurement unit 400. The interface unit 304 is typically an USB interface. The analysis unit 300 contains a controller 301, a bus 302, a storage unit 303, an interface unit 304, a display 305, and an operation unit 306. The analysis unit 300 is typically constituted by a personal computer (see the analysis unit 300 in Fig. 1), and executes a program product stored in the storage unit 303, to control the measurement unit 400 of the analysis system 500. The analysis unit 300 analyzes data that includes the optical information acquired by the measurement unit 400, and displays an analysis result on the display 305. The analysis unit 300 may also classify the cells with reference to the optical information.

The storage unit 303 typically stores a program product for controlling the measurement unit 400, and a program product for analyzing data acquired by the measurement unit 400. The display 305 typically displays result of analysis of data acquired by the measurement unit 400. The operation unit 306 has a keyboard, and a pointing device that contains a mouse or a touch panel.

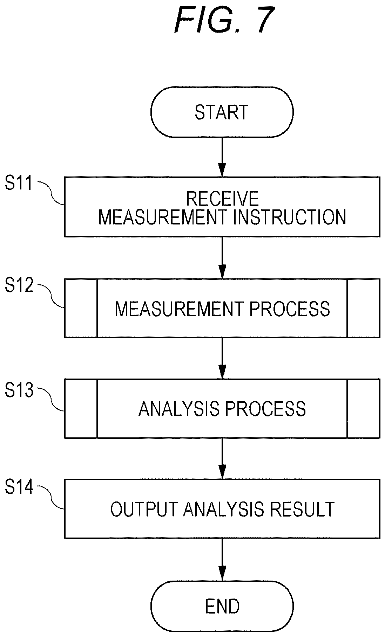

Exemplary operations of the analysis system 500 will now be described while referring to Fig. 7, without limiting the invention. In step S 11, the controller 301 of the analysis unit 300 receives a measurement start instruction from the user, through the operation unit 306. Upon receiving the measurement start instruction, the controller 301 transmits instruction data for instructing the measurement start to the measurement unit 400. The measurement unit 400 receives the instruction data, and executes a measurement process in step S12.

The method for analyzing blood cells of this embodiment typically encompasses a first embodiment and a second embodiment below. In the "first embodiment", a measurement sample prepared under hemolytic conditions is measured, and white blood cell in the measurement sample is classified. In the "second embodiment", the measurement process and the analysis process will vary, depending on a target to be measured designated by the user. With white blood cell designated, the measurement process and the analysis process will take place in the same manner as in the first embodiment. With reticulocyte and platelet designated, the measurement sample prepared under non-hemolytic conditions is measured, and reticulocyte and platelet are classified from the particles in the measurement sample.

### (Measurement Process in First Embodiment)

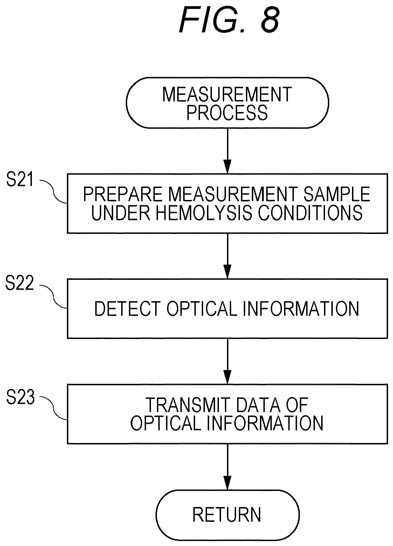

Exemplary measurement process in step S12 in Fig. 7 according to the first embodiment will be explained while referring to Fig. 8, without limiting the invention. In step S21, the controller 301 of the analysis unit 300 instructs the measurement unit 400 to prepare the measurement sample from the specimen, under hemolytic conditions. More specifically, the measurement unit 400 mixes the specimen, the staining reagent, and the hemolysis reagent in the reaction chamber 420, to prepare the measurement sample. In step S22, the controller 301 instructs the measurement unit 400 to feed the measurement sample to the FCM detector 460, and to implement optical measurement by flow cytometry. The measurement unit 400 thus detects optical information of each particle in the measurement sample, typically including waveform data of SSC-1, SSC-2, FSC-2, and SFL-1. In step S23, the controller 301 instructs the measurement unit 400 to transmit the optical information to the analysis unit 300. The measurement process thus finishes. The analysis unit 300 then implements the analysis process in step S13 in Fig. 7.

### (Measurement Process in Second Embodiment)

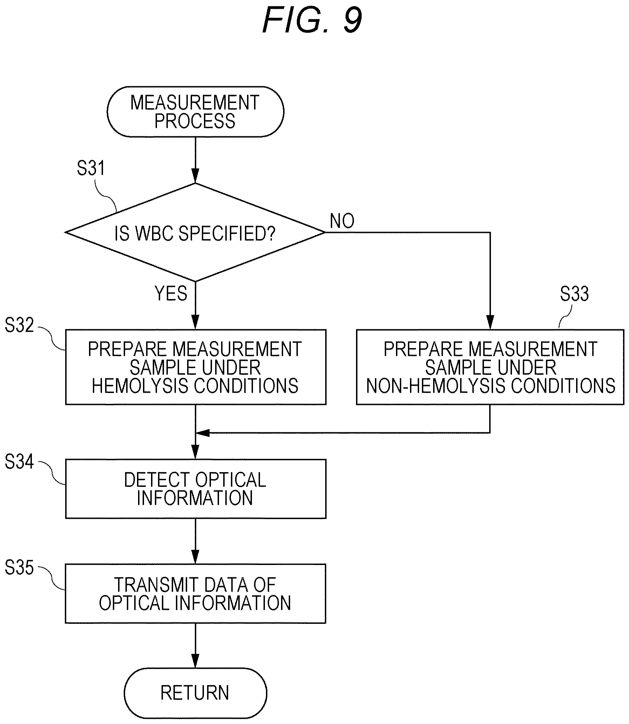

Exemplary measurement process in step S12 in Fig. 7 according to the second embodiment will be explained, while referring to Fig. 9, without limiting the invention. In step S31, the controller 301 of the analysis unit 300 receives a measurement start instruction from the user, through the operation unit 306. The target to be measured corresponds to any type of blood cell to be analyzed, which is exemplified by white blood cell, reticulocyte, and platelet. Input for designating the target to be measured is acceptable, typically through a screen for selecting the target to be measured, displayed on the display 305. More specifically, the controller 301 displays a selection screen on which the target to be measured is designated, on the display 305. On the selection screen, the user can designate the target to be measured, which is white blood cell, or, reticulocyte and platelet. After designating the target to be measured, and upon user's decision regarding the measurement start made on the screen, the measurement takes place. Upon acceptance, by the controller 301 in step S31, of a designation of white blood cell as the target to be measured, the controller 301 then causes in step S32 the measurement unit 400 to prepare the measurement sample from the specimen under hemolytic conditions. Preparation of the measurement sample under hemolytic conditions is as described previously. Meanwhile, upon acceptance, by the controller 301 in step S31, of a designation of reticulocyte and platelet as the target to be measured, the controller 301 then causes in step S33 the measurement unit 400 to prepare the measurement sample from the specimen under non-hemolytic conditions. More specifically, the measurement unit 400 mixes the specimen, the staining reagent, and the dilution reagent in the reaction chamber 420, to prepare the measurement sample. The hemolysis reagent is not used under the non-hemolytic conditions. Steps S34 and S35 are the same as steps S22 and S23, respectively. After completion of the measurement process, the analysis unit 300 then implements the analysis process in step S13 in Fig. 7. Exemplary analysis processes of the individual embodiments will be described later.

### (Specimen)

The specimen is blood specimen. The blood specimen is exemplified by whole blood, and dilution of the whole blood. The whole blood is typically peripheral blood collected from the subject. The blood specimen may contain an anticoagulant. The anticoagulants is exemplified by ethylenediaminetetraacetate (EDTA), EDTA salts (EDTA·2K, EDTA 2Na, for example), sodium citrate, heparin, and warfarin. The dilution of whole blood is obtainable typically by diluting the whole blood with a suitable aqueous solvent, preferably with a dilution reagent described later. The aqueous solvent is exemplified by water, physiological saline, and aqueous solution of buffering agent.

The particles in the measurement sample are derived from blood components of the specimen. As referred herein, the "blood components" encompass the elements having been known to reside in blood, and abnormal cell. The elements having been known to reside in blood are typically blood cells usually contained in peripheral blood of healthy person. Such blood cells are exemplified by white blood cell, mature red blood cell, reticulocyte, and platelet. The "mature red blood cell" is a terminally differentiated red blood cell having neither nucleus nor nucleic acid. The term "mature red blood cell" herein is used for distinguishing this cell from reticulocyte. The mature red blood cell and reticulocyte, if not needed to be distinguished herein, may be collectively referred to as "red blood cell" or "RBC". The "reticulocyte" is a young red blood cell derived from erythroblastic cell in bone marrow, which is immediately after enucleated and released into peripheral blood. The reticulocyte usually becomes mature red blood cell in approximately one day. The reticulocyte contains therein a small amount of ribonucleic acid, and organelles such as ribosome and mitochondria, all of which are not found in mature red blood cell. The term "platelet" herein encompasses immature platelet and large platelet. "Immature platelet", also referred to as reticulated platelet, is a young platelet that has just been released into the peripheral blood. The mature platelet does not contain nucleic acid, whereas the immature platelet contains a small amount of ribonucleic acid. The "large platelet" has a diameter of 4 µm or larger. The platelet is usually 2 to 3 µm in diameter.

The "abnormal cell" refers to a cell that does not normally appear in blood. The abnormal cell is exemplified by immature granulocyte. The immature granulocyte is a progenitor cell of granulocyte, and is exemplified by promyelocyte, myelocyte, and metamyelocyte. The immature granulocyte, usually found in bone marrow, may occasionally appear in blood, typically due to myeloid leukemia, bone metastasis, or severe infection. In a case where immature granulocyte is possibly contained in the blood specimen, the term "white blood cell" also encompasses the immature granulocyte. The abnormal cell also encompasses non-cellular particle and microorganism. The non-cellular particle is exemplified by platelet aggregation, red blood cell ghost, and lipid particle. The microorganism is exemplified by bacteria and fungi. The platelet aggregation occurs in a blood collection tube, due to contamination with tissue fluid during blood collection, insufficient mixing, or action of EDTA.

### (Hemolysis Reagent)

The hemolysis reagent used for preparing the measurement sample will be described. The hemolysis reagent is a reagent for lysing red blood cell in the specimen, upon mixed with the specimen. The hemolysis reagent contains a surfactant, and is used in combination with a staining reagent that contains a fluorescent dye. The surfactant can hemolyze red blood cells in the specimen, and can cause damage to cell membrane of cells other than red blood cell, to an extent enough to allow the fluorescent dye to pass therethrough. The surfactant is exemplified by nonionic surfactant, cationic surfactant, and combinations thereof. The hemolysis reagent preferably contains the nonionic surfactant.

The nonionic surfactant is exemplified by those represented by formula (II) below:

R₁-R₂-(CH₂CH₂O)ₙ-H (II)

(where, R₁ represents an alkyl group, an alkenyl group or an alkynyl group having 8 or more and 25 or less carbon atoms, and R₂ represents an oxygen atom, -(COO)- or a group represented by formula (III) below: 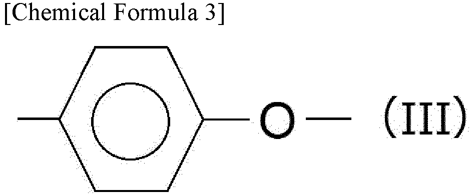 where, n represents an integer of 10 or larger and 50 or smaller).

Concentration of the nonionic surfactant represented by formula (II), in the hemolysis reagent, is typically 0.5 g/L or higher and 5 g/L or lower. The nonionic surfactant represented by formula (II) is specifically exemplified by polyoxyethylene alkyl ether, polyoxyethylene sterol, polyoxyethylene castor oil, polyoxyethylene sorbitol fatty acid ester, polyoxyethylene alkylamine, polyoxyethylene polyoxypropylene alkyl ether, and combinations thereof. Among them, polyoxyethylene alkyl ether is preferred. Only one kind, or two or more kinds of the nonionic surfactant may be contained in the hemolysis reagent. The hemolysis reagent may further contain a nonionic surfactant, besides the nonionic surfactant represented by formula (II).

The hemolysis reagent may further contain a cationic surfactant. The cationic surfactant is exemplified by quaternary ammonium salt-type surfactant, pyridinium salt-type surfactant, and combinations thereof. The quaternary ammonium salt-type surfactant suitably used herein is preferably a surfactant having 9 to 30 carbon atoms in total, represented by formula (IV) below. Only one kind, or two or more kinds of the cationic surfactant may be contained in the hemolysis reagent.

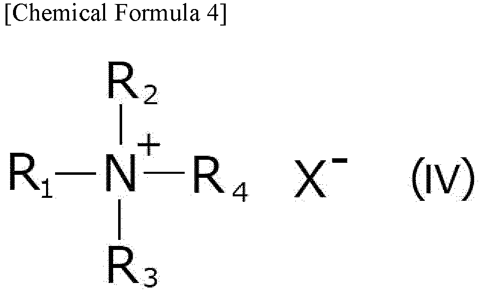

In the formula (IV), R₁ represents an alkyl group or an alkenyl group having 6 to 18 carbon atoms; each of R₂ and R₃, identically or differently, represents an alkyl group having 1 to 4 carbon atoms, or an alkenyl group; R₄ represents an alkyl group having 1 to 4 carbon atoms, an alkenyl group, or a benzyl group; and X⁻ represents a halogen ion. The "halogen" is fluorine, chlorine, bromine, or iodine.

In formula (IV), R₁ preferably represents an alkyl group or an alkenyl group having 6, 8, 10, 12, or 14 carbon atoms. A linear alkyl group is particularly preferred. R₁ is more specifically exemplified by an octyl group, a decyl group, or a dodecyl group. Preferably, each of R₂ and R₃, identically or differently, represents a methyl group, an ethyl group, or a propyl group. R₄ preferably represents a methyl group, an ethyl group, or a propyl group.

The pyridinium salt-type surfactant is typically exemplified by a surfactant represented by formula (V):

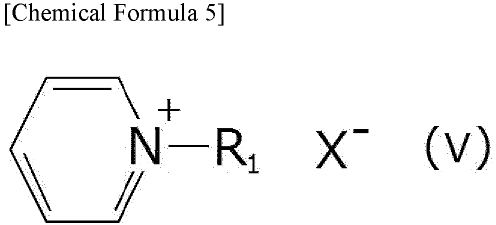

In formula (V), R₁ represents an alkyl group or an alkenyl group having 6 to 18 carbon atoms; and X⁻ represents a halogen ion.

In formula (V), R₁ preferably represents an alkyl group or an alkenyl group having 6, 8, 10, 12, or 14 carbon atoms. A linear alkyl group is particularly preferred. R₁ is more specifically exemplified by an octyl group, a decyl group, or a dodecyl group.

Concentration of the cationic surfactant in the hemolysis reagent is suitably selectable, depending on types of the surfactant. Concentration of the cationic surfactant in the hemolysis reagent is typically 10 ppm or higher and 10,000 ppm or lower.

The hemolysis reagent may contain a buffering agent for keeping pH constant. The buffering agent is exemplified by inorganic acid salt, organic acid salt, Good's buffer, and combinations thereof. The inorganic acid salt is exemplified by phosphate, borate, and combinations thereof. The organic acid salt is exemplified by citrate, malate, and combinations thereof. The Good's buffer is exemplified by MES, Bis-Tris, ADA, PIPES, Bis-Tris-Propane, ACES, MOPS, MOPSO, BES, TES, HEPES, HEPPS, Tricine, Tris, Bicine, TAPS, and combinations thereof.

The hemolysis reagent may further contain an aromatic organic acid. Concentration of the aromatic organic acid in the hemolysis reagent is not particularly limited. The aromatic organic acid herein means acid that has at least one aromatic ring in the molecule, and salt thereof. The aromatic organic acid is exemplified by aromatic carboxylic acid, and aromatic sulfonic acid. The aromatic carboxylic acid is exemplified by phthalic acid, benzoic acid, salicylic acid, hippuric acid, salts thereof, and combinations thereof. The aromatic sulfonic acid is exemplified by p-aminobenzenesulfonic acid, benzenesulfonic acid, salts thereof, and combinations thereof. Only one kind, or two or more kinds of the aromatic organic acid may be contained in the hemolysis reagent. Some aromatic organic acid can occasionally demonstrate a buffering function. When using the aromatic organic acid with the buffering function, use of the buffering agent is optional. The aromatic organic acid may be combined with the buffering agent.

The hemolysis reagent is preferably a liquid reagent. A solvent is not particularly limited as long as it can dissolve the individual components such as the aforementioned surfactant. The solvent is exemplified by water, organic solvent, and mixtures thereof. The organic solvent is preferably a water-miscible solvent, and is exemplified by alcohol having 1 to 6 carbon atoms, ethylene glycol, diethylene glycol, polyethylene glycol, and DMSO.

pH of the hemolysis reagent is not particularly limited. The pH of the hemolysis reagent is typically 4.5 or higher, and preferably 5 or higher. The pH of the hemolysis reagent is typically 11 or lower, and preferably 10 or lower. The pH is adjustable with use of any of known bases (such as sodium hydroxide) or acids (such as hydrochloric acid).

The hemolysis reagent may have an osmotic pressure not particularly limited, which is preferably 150 mOsm/kg·H₂O or lower from the viewpoint of hemolysis efficiency of red blood cell. The osmotic pressure may be adjusted by adding an appropriate osmoregulator. The osmoregulator is exemplified by sugar, amino acid, organic solvent, sodium chloride, and combinations thereof.

The hemolysis reagent usable herein may be any of commercially available hemolysis reagents for blood cell analysis. Such reagents are exemplified by Lysercell WDF (Sysmex Corporation) and Lysercell WDF II (Sysmex Corporation).

### (Dilution Reagent)

The dilution reagent used for preparing the measurement sample will be described. The dilution reagent preferably contains a solution of the buffering agent. Preferred example of the solvent includes water and physiological saline. Water is particularly preferred. The buffering agent preferably demonstrates a buffering function within a pH range from 6 or higher and 11 or lower. Such buffers is selectable typically from carboxylate, phosphate, Good's buffer, taurine, and triethanolamine. The dilution reagent typically has a pH of 6 or higher and 11 or lower, which is preferably 7 or higher and 10 or lower, and more preferably 8 or higher and 9.5 or lower. With the dilution reagent whose pH is 6 or above, red blood cell will be less likely to hemolyze in the measurement sample. This successfully suppresses red blood cell ghost from occurring. With the dilution reagent whose pH is 11 or below, red blood cells and red blood cell ghost may be prevented from being non-specifically stained with the fluorescent dye. A preferred dilution reagent is an aqueous solution of the buffering agent, having a pH of 6 or higher and 11 or lower.

The dilution reagent may further contain, besides the buffering agent, components such as osmotic pressure compensator, staining accelerator, highly-charged anion, and preservative. The osmotic pressure compensator is a substance capable of maintaining osmotic pressure of the dilution reagent within an appropriate range. The osmotic pressure compensator is exemplified by alkali metal salt of organic acid such as propionic acid; saccharide such as glucose or mannose; alkali metal halide such as sodium chloride; and alkaline earth metal halide such as magnesium chloride. The osmotic pressure compensator may be used singly or in combination of two or more kinds thereof. The osmotic pressure compensator, when used, is added to the dilution reagent, so as to adjust the osmotic pressure of the dilution reagent preferably to 150 mOsm/kg■H₂O or higher and 600 mOsm/kg■H₂O or lower.

The staining accelerator is a substance capable of promoting permeability of the fluorescent dye into the blood cells. The staining accelerator is exemplified by surfactant, which is preferably cationic surfactant. Quaternary ammonium salt type surfactant is particularly preferred as the cationic surfactant, which is exemplified by decyltrimethylammonium bromide (DTAB), lauryltrimethylammonium chloride (LTAC), octyltrimethylammonium bromide, cetyltrimethylammonium chloride, and myristyltrimethylammonium bromide. The cationic surfactant may be used singly or in combination of two or more kinds thereof.

The concentration of the cationic surfactant in the dilution reagent is not particularly limited as long as hemolysis of red blood cell may be suppressed. The concentration may be appropriately determined typically within the range from 50 ppm or higher and 20,000 ppm or lower. In a specific case of using DTAB, the concentration thereof in the dilution reagent is preferably 500 ppm or higher and 3000 ppm or lower. In a case of using LTAC, the concentration thereof in the dilution reagent is preferably 100 ppm or higher and 500 ppm or lower. With such concentration of the cationic surfactant contained therein, the dilution reagent can suppress hemolysis of red blood cell, and can promote staining of the blood cells with the fluorescent dye.

The highly-charged anion is added to the dilution reagent, in order to suppress non-specific staining of red blood cell with the fluorescent dye. The highly-charged anion is exemplified by sulfate ion, phosphate ion, carbonate ion, and polycarboxylate ion. Compounds capable of supplying these ions are exemplified by citric acid, sulfuric acid, phosphoric acid, EDTA, and alkali metal salts thereof. The highly-charged anion may be used singly or in combination of two or more kinds thereof.

The preservative contained in the dilution reagent is exemplified by sodium 2-pyridylthio-1-oxide and β-phenethyl alcohol.

The method for analyzing blood cells of this embodiment may employ a commercially available dilution reagent. The commercially available dilution reagent is exemplified by CELLPACK (registered trademark) DFL (Sysmex Corporation).

### (Staining Reagent and Fluorescent Dye)

Staining reagent used for preparing the measurement sample will be described. The fluorescent dye suitable for the staining reagent is a fluorescent dye represented by formula (I) above. This fluorescent dye is a cyanine-based fluorescent dye that can be excited by the light of the first wavelength, and can bind to nucleic acid. With such binding property to nucleic acid, the fluorescent dye makes it possible to distinguish nucleic acid-free cells such as mature red blood cell and platelet, from nucleic acid-containing cells such as reticulocyte and white blood cells. Since the fluorescent dye represented by formula (I) above has a maximum absorption wavelength fallen in the range of the first wavelength, so that the fluorescence from this fluorescence dye may be suppressed from interfering with the scattered light that corresponds to the light of the second wavelength.

The "alkyl group" can be either linear or branched. The alkyl group having 1 to 18 carbon atoms, represented by R₁ to R₄ and R₆ to R₈ in formula (I), is exemplified by methyl, ethyl, propyl, isopropyl, t-butyl, n-butyl, isopentyl, neopentyl, t-pentyl, isohexyl, heptyl, octyl, decyl, dodecyl, tetradecyl, hexadecyl, and octadecyl. The alkyl group preferably has 1 to 10 carbon atoms, in which the number of carbon atoms is more preferably 1 to 6, and is particularly 1 to 3. Preferred examples of the alkyl group include methyl, ethyl, propyl, isopropyl, t-butyl, n-butyl, isopentyl, neopentyl, t-pentyl, and isohexyl. The alkyl group represented by R₅ in formula (I) preferably has 1 to 3 carbon atoms.

The "alkenyl group" can be either linear or branched. The alkenyl group having 3 to 20 carbon atoms, represented by R₁ and R₂ in formula (I), is exemplified by allyl, propenyl, isopropenyl, butenyl, isobutenyl, pentenyl, isopentenyl, hexenyl, heptenyl, octenyl, decenyl, dodecenyl, tetradecenyl, hexadecenyl, octadecenyl, and icosenyl. The alkenyl group preferably has 3 to 14 carbon atoms, in which the number of carbon atoms is more preferably 3 to 10, and is particularly 3 to 6.

The substituent of the phenyl group is exemplified by -NH₂, halogen, -OH, - SH, -CN, -NO₂, -COOH, and alkyl group having 1 to 6 carbon atoms. The substituent on the benzyl group is exemplified by -CN, -COOH, -NH₂, -NO₂, -OH, -SH, alkyl group having 1 to 18 carbon atoms, alkoxy group having 1 to 6 carbon atoms, alkylamino group having 1 to 6 carbon atoms, acylamino group having 1 to 6 carbon atoms, halogen, and haloalkyl group having 1 to 6 carbon atoms. "Haloalkyl group" is an alkyl group having at least one hydrogen substituted with halogen. In the haloalkyl group having two or more halogens, types of such halogens may be same or different.

In formula (I) above, with either X or Y given by an oxygen atom, the other preferably represents a sulfur atom, a selenium atom, or C(CH₃)₂. The counter ion Z⁻ in formula (I) is exemplified by F⁻, Cl⁻, Br⁻, I⁻, CF₃SO₃⁻, BF₄⁻, and ClO₄⁻.

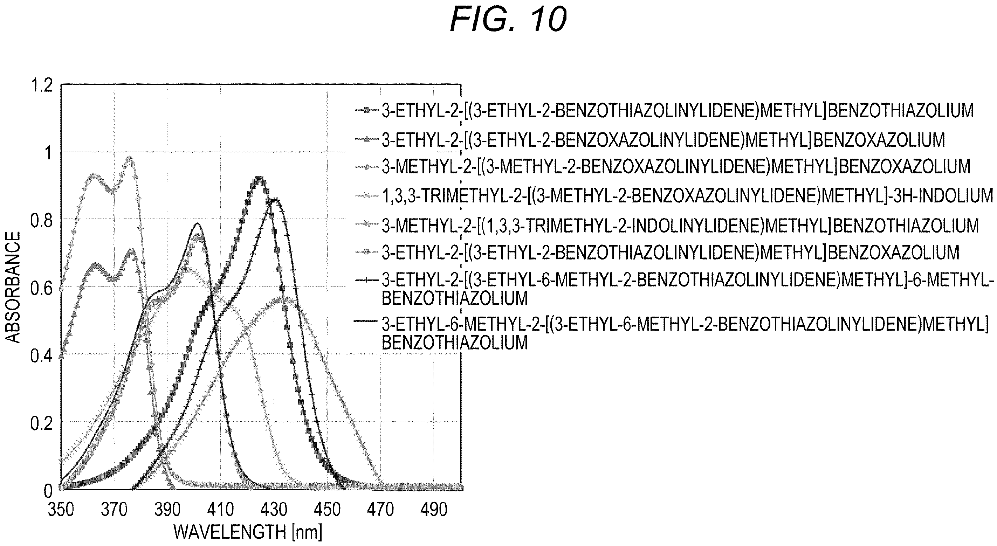

The fluorescent dye represented by formula (I) is exemplified by 3-methyl-2-[(3-methyl-2-benzoxazolinylidene)methyl]benzoxazolium, 3-ethyl-2-[(3-ethyl-2-benzothiazolinylidene)methyl]benzothiazolium, 3-ethyl-2-[(3-ethyl-6-methyl-2-benzothiazolinylidene)methyl]-6-methyl-benzothiazolium, 3-ethyl-2-[(3-ethyl-2-benzoxazolinylidene)methyl]benzoxazolium, 3-ethyl-2-[(3-ethyl-2-benzothiazolinylidene)methyl]benzoxazolium, 3-ethyl-6-methyl-2-[(3-ethyl-6-methyl-2-benzothiazolinylidene)methyl]benzothiazolium, 3-methyl-2-[(1,3,3-trimethyl-2-indolinylidene)methyl]benzothiazolium, and 1,3,3-trimethyl-2-[(3-methyl-2-benzoxazolinylidene)methyl]-3H-indolium. Absorption spectra of these fluorescent dyes in ethanol are illustrated in Fig. 10. Maximum absorption wavelengths (λmax) of these fluorescent dyes are summarized in Table 1.

**[Table 1]**

| Fluorescence dye | λmax (nm) |
|---|---|
| 3-ethyl-2-[(3-ethyl-2-benzothiazolinylidene)methyl]benzothiazolium | 424 |
| 3-ethyl-2-[(3-ethyl-2-benzoxazolinylidene)methyl]benzoxazolium | 376 |
| 3-methyl-2-[(3-methyl-2-benzoxazolinylidene)methyl]benzoxazolium | 376 |
| 1,3,3-trimethyl-2-[(3-methyl-2-benzoxazolinylidene)methyl]-3H-indolium | 398 |
| 3-methyl-2-[(1,3,3-trimethyl-2-indolinylidene)methyl]benzothiazolium | 434 |
| 3-ethyl-2-[(3-ethyl-2-benzothiazolinylidene)methyl]benzoxazolium | 402 |
| 3-ethyl-2-[(3-ethyl-6-methyl-2-benzothiazolinylidene)methyl]-6-methyl-benzothiazolium | 430 |
| 3-ethyl-6-methyl-2-[(3-ethyl-6-methyl-2-benzothiazolinylidene)methyl]benzothiazolium | 401 |

Preferred fluorescent dyes include 3-ethyl-2-[(3-ethyl-2-benzothiazolinylidene)methyl]benzothiazolium, 3-ethyl-2-[(3-ethyl-6-methyl-2-benzothiazolinylidene)methyl]-6-methyl-benzothiazolium, 3-ethyl-2-[(3-ethyl-2-benzothiazolinylidene)methyl]benzoxazolium, 3-ethyl-6-methyl-2-[(3-ethyl-6-methyl-2-benzothiazolinylidene)methyl]benzothiazolium, 3-methyl-2-[(1,3,3-trimethyl-2-indolinylidene)methyl]benzothiazolium, and 1,3,3-trimethyl-2-[(3-methyl-2-benzoxazolinylidene)methyl]-3H-indolium.

The staining reagent preferably contains a solvent of the fluorescent dye. The solvent is not particularly limited as long as it can dissolve the fluorescent dye. The solvent is exemplified by water, organic solvent, and mixtures thereof. The organic solvent is exemplified by alcohol having 1 to 6 carbon atoms, ethylene glycol, diethylene glycol, polyethylene glycol, and dimethyl sulfoxide (DMSO). Concentration of the fluorescent dye in the staining reagent may be suitably determined, depending on types of the fluorescent dye. The concentration of the fluorescent dye is typically 100 ppm or higher, preferably 500 ppm or higher, and more preferably 1000 ppm or higher. Concentration of the fluorescent dye is typically 100000 ppm or lower, preferably 50000 ppm or lower, and more preferably 10000 ppm or lower.

Since the fluorescent dye itself can bind to the nucleic acid of the cell as described above, the method for analyzing blood cells of this embodiment no longer needs staining of the cells with use of an antibody labeled with the fluorescent dye. Hence in the method for analyzing blood cells of this embodiment, the fluorescent dye does not contain an antibody.

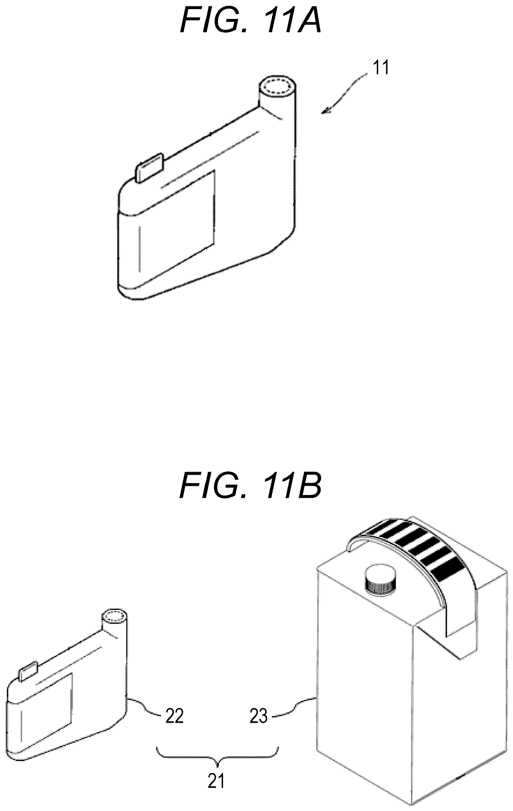

The staining reagent that contains the fluorescent dye may also be provided as a reagent for blood cell analysis, used for the method for analyzing blood cells of this embodiment. The reagent for blood cell analysis of this embodiment may typically have a form such that a solution of the fluorescent dye is filled in a container. Referring now to Fig. 11A, reference sign 11 denotes a container, having the reagent for blood cell analysis of this embodiment filled therein. The container 11, when enclosed in a package box, may be typically accompanied by a package insert that describes composition of the reagent for blood cell analysis, directions for use, storage and so forth, and a cushion material for cushioning external impact, enclosed therein.

The staining reagent that contains the fluorescent dye, and the hemolysis reagent may also be provided in the form of a reagent kit for blood cell analysis, used for the method for analyzing blood cells of this embodiment. The reagent for blood cell analysis of this embodiment may typically be in the form such that a solution of the fluorescent dye, and the hemolysis reagent are individually filled in containers. Referring now to Fig. 11B, reference sign 21 denotes a reagent kit for blood cell analysis of this embodiment, reference sign 22 denotes a first container that contains a solution of the fluorescent dye, and reference sign 23 denotes a second container that contains the hemolysis reagent. The reagent kit for blood cell analysis of this embodiment may be enclosed in a package box. The reagent kit 21, when enclosed in the package box, may be typically accompanied by a package insert and cushion material enclosed therein. The reagent kit of this embodiment may further contain, for example, the dilution reagent or a calibrator.

A further embodiment relates to use of the fluorescent dye, for manufacture of the reagent for blood cell analysis. Details of the fluorescent dye and the reagent for blood cell analysis are as described above. A further embodiment relates to use of the fluorescent dye and the hemolysis reagent, for manufacture of the reagent kit for blood cell analysis. Details of the fluorescent dye, the hemolysis reagent, and the reagent kit for blood cell analysis are as described above.

### (Preparation of Measurement Samples under Hemolytic Conditions)

The fluorescent dye represented by formula (I) above is mixed with the specimen and the dilution reagent, so that the concentration (final concentration) in the measurement sample falls within a predetermined range. A preferred final concentration of the fluorescent dye in the measurement sample is 1000 ppm or lower, which is more preferably 100 ppm or lower, and even more preferably 10 ppm or lower. A preferred final concentration of the fluorescent dye in the measurement sample is 0.001 ppm or higher, which is more preferably 0.01 ppm or higher, and even more preferably 0.1 ppm or higher.

A ratio of mixing of the hemolysis reagent, the staining reagent, and the specimen may be appropriately determined, according to the concentration of the fluorescent dye in the staining reagent, or to the types of the specimen. The ratio of mixing of the hemolysis reagent, the staining reagent, and the specimen is, for example, preferably 1000 : (1 or above) : (1 or above) on the volume basis. The ratio is more preferably 1000 : (10 or above) : (10 or above), and even more preferably 1000 : (15 or above) : (15 or above). The ratio of mixing of the hemolysis reagent, the staining reagent, and the specimen is preferably, for example, 1000 : (50 or below) : (50 or below) on the volume basis. The ratio is more preferably 1000 : (30 or below) : (30 or below), and even more preferably 1000 : (25 or below) : (25 or below). The ratio of mixing of the staining reagent and the specimen may be the same or different.

Red blood cell is the most abundant blood cell in the blood specimen. Since the hemolysis reagent can lyse red blood cell, so that use thereof in analysis of blood cell which is not lysed with the hemolysis reagent (white blood cell, for example) can exclude influence of red blood cell. Use of the hemolysis reagent can also promote staining of white blood cell with the fluorescent dye, with the aid of the surfactant contained therein. As can be seen, preparation of the measurement sample under hemolytic conditions is suitable for the analysis of white blood cell.

### (Preparation of Measurement Samples under Non-Hemolytic Conditions)

The fluorescent dye represented by formula (I) above is mixed with the specimen and the dilution reagent, so that the final concentration falls within a predetermined range. A preferred final concentration of the fluorescent dye in the measurement sample is 500 ppm or lower, which is more preferably 100 ppm or lower, and even more preferably 50 ppm or lower. A preferred final concentration of the fluorescent dye in the measurement sample is 0.01 ppm or higher, which is more preferably 0.1 ppm or higher, and even more preferably 1 ppm or higher.

The ratio of mixing of the dilution reagent, the staining reagent, and the specimen may be appropriately determined, according to the concentration of the fluorescent dye in the staining reagent, or types of the specimen. The ratio of mixing of the hemolytic reagent, the staining reagent, and the specimen is preferably, for example, 1000 : (0.1 or above) : (1 or above) on the volume basis. The ratio is more preferably 1000 : (0.5 or above) : (5 or above), and even more preferably 1000 : (1 or above) : (10 or above). The ratio of mixing of the dilution reagent, the staining reagent, and the specimen is preferably, for example, 1000 : (50 or below) : (100 or below) on the volume basis. The ratio is more preferably 1000 : (30 or below) : (50 or below), and even more preferably 1000 : (20 or below) : (25 or below). The ratio of mixing of the staining reagent and the specimen may be the same or different.

Since reticulocyte is lysed with the hemolysis reagent similarly to mature red blood cell, so that preparation of the measurement sample under non-hemolytic conditions is suitable for the analysis of reticulocyte. The prior method, although capable of classifying and counting reticulocyte and platelet by a one-time measurement, has occasionally suffered from poor accuracy of acquired platelet count, depending on specimens. An additional measurement has therefore been necessary to count platelet more accurately, under conditions suited to the platelet measurement. In contrast, the method for analyzing blood cells of this embodiment can classify and count reticulocyte and platelet more accurately, with a one-time measurement of the measurement sample prepared under non-hemolytic conditions. As can be seen, preparation of the measurement sample under non-hemolytic conditions is suitable for the analysis of reticulocyte and platelet. When classifying reticulocyte and platelet by the method for blood cell analysis of this embodiment, the measurement sample does not contain the hemolysis reagent. That is, the measurement sample is prepared without going through a process for hemolyzing red blood cell.

A further embodiment relates to a method for fluorescently staining blood cells, including contacting the fluorescent dye represented by formula (I) above, with the blood cells. This method can stain the blood cells, by mixing the fluorescent dye, with a specimen that contains the blood cells. The method may also use the aforementioned staining reagent, as the fluorescent dye. In the mixing of the fluorescent dye with the specimen, the hemolysis reagent or the dilution reagent may be further mixed. Concentrations and ratios of mixing of the fluorescent dye and the individual reagents in the mixing are the same as those described for the preparation of the measurement sample. Details of the fluorescent dye, the specimen, the hemolysis reagent, the dilution reagent, and the measurement sample are as described previously.

### (Analysis Process in First Embodiment)

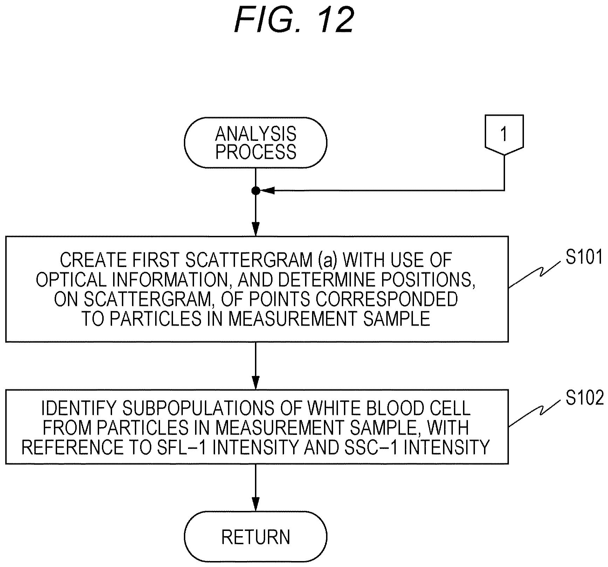

Exemplary analysis process in step S13 in Fig. 7 according to the first embodiment will be explained, while referring to Figs. 12 and 13, without limiting the invention. White blood cell will also be denoted as "WBC", mature red blood cell as "mRBC", and reticulocyte as "RET".

Referring now to Fig. 12, the analysis process in the first embodiment classifies WBC in the measurement sample into subpopulations. In the first embodiment, the measurement sample, prepared with use of the hemolysis reagent, contains a red blood cell ghost which is produced as a result of hemolysis of mRBC and RET. WBC is classified into subpopulations by identifying the individual subpopulations of WBC from the particles in the measurement sample, with reference to the fluorescence information and the first scattered light information, and/or, the fluorescence information and the second scattered light information. This example employs the first side-fluorescence intensity (also referred to as "SFL-1 intensity") as the fluorescence information, the first side-scattered light intensity (also referred to as "SSC-1 intensity") as the first scattered light information, and the second side-scattered light intensity (also referred to as "SSC-2 intensity") as the second scattered light information.

WBC may be classified into subpopulations, typically by analysis on scattergrams. A scattergram for classifying WBC into subpopulations with reference to the fluorescence information and the first scattered light information is also referred to as "first scattergram (a)". A scattergram for classifying WBC into subpopulations with reference to the fluorescence information and the second scattered light information is also referred to as "first scattergram (b)". In the analysis process of the first embodiment, either the first scattergram (a) or (b) is created. Alternatively, both the first scattergrams (a) and (b) may be created.

Referring now to Fig. 12, the analysis unit 300 in step S101 creates the first scattergram (a), with reference to the acquired optical information. The first scattergram (a) has the SSC-1 intensity and the SFL-1 intensity plotted on two coordinate axes. In step S101, the analysis unit 300 may alternatively create the first scattergram (b), with reference to the acquired optical information. The first scattergram (b) has the SSC-2 intensity and the SFL-1 intensity plotted on two coordinate axes. The abscissa and the ordinate of the first scattergrams (a) and (b) may be expressed in either logarithmic or linear scale. Preferably, the abscissa of the first scattergrams (a) and (b) is given in a logarithmic scale, meanwhile the ordinate in a linear scale. Step S101 determines positions of points corresponded to the individual particles in the measurement sample, on the thus created first scattergram (a). In an alternative case where the first scattergram (b) was created, positions of points corresponded to the individual particles in the measurement sample are determined on the first scattergram (b).

In step S102, the analysis unit 300 identifies the individual subpopulations of white blood cell from the particles in the measurement sample, with reference to the thus determined SFL-1 intensity, and the SSC-1 intensity or the SSC-2 intensity of the individual points. At least the lymphocyte population, the monocyte population, and the granulocyte population are determined as the subpopulations. As described previously, granulocyte includes neutrophil, eosinophil, and basophil. If possible, granulocyte is further classified to identify at least a neutrophil population, an eosinophil population, and a basophil population. Preferably, the lymphocyte population, the monocyte population, the neutrophil population, and the eosinophil population are identified from the particles in the measurement sample. More preferably, the lymphocyte population, the monocyte population, the neutrophil population, the eosinophil population, and the basophil population are identified from the particles in the measurement sample. Also a population of immature granulocytes, when contained in the measurement sample, may be identified, besides the individual subpopulations of white blood cell. An algorithm per se for identifying the individual subpopulations of white blood cell has been known. For example, white blood cell can be classified into the subpopulations, with use of a program product stored in the storage unit 303 of the analysis unit 300. Alternatively, since the positions where the individual subpopulations of white blood cell appear on the scattergram are already known, so that the individual subpopulations of white blood cell may be identified with reference to the scattergram. Fig. 13 illustrates exemplary first scattergrams (a) and (b) on which white blood cell is classified into three types or five types of subpopulations. An area surrounded by the broken line in each scattergram indicates an area where immature granulocyte appears. In the drawings, "Lymp" stands for the lymphocyte population, "Mono" for the monocyte population, "Gran" for the granulocyte population, "Neut" for the neutrophil population, "Eo" for the eosinophil population, and "Baso" for the basophil population. "IG" stands for immature granulocytes.

Referring now to (A) and (B) in Fig. 13, three populations of lymphocyte, monocyte, and granulocyte appear to distribute as the individual subpopulations of white blood cell, on the first scattergrams (a) and (b). Also debris such as red blood cell ghost appears in a region where the SFL-1 intensity is low. As can be seen from these drawings, whichever of the first scattergrams (a) and (b) can classify white blood cell into three subpopulations of lymphocyte, monocyte, and granulocyte. The immature granulocyte, when contained in the measurement sample, appears in an area where the SFL-1 intensity is higher than that of the granulocyte population, and the SSC-1 intensity or the SSC-2 intensity is higher than that of the monocyte population, as indicated by the broken line.

If granulocyte can be classified into subpopulations, then white blood cell can be classified typically into five subpopulations. Referring now to (C) and (D) in Fig. 13, five populations of lymphocyte, monocyte, neutrophil, eosinophil, and basophil distribute as the subpopulations of white blood cell, on both of the first scattergrams (a) and (b). Immature granulocytes, when contained in the measurement sample, appears in an area where the fluorescence intensity is higher than that of the neutrophil population, and the SSC-1 intensity or the SSC-2 intensity is higher than that of the monocyte population, as indicated by the broken line. White blood cell exemplified in Fig. 13 is classified into, but not limited to, three or five subpopulations. White blood cell may alternatively be classified into four subpopulations of lymphocyte, monocyte, neutrophil, and eosinophil. Cells contained in the individual subpopulations may be optionally counted. The cells are counted with use of the analysis unit 300. After completion of the classification of white blood cell, the analysis unit 300 finishes the analysis process. The process then advances to step S14 in Fig. 7.

### (Analysis Process of Second Embodiment)

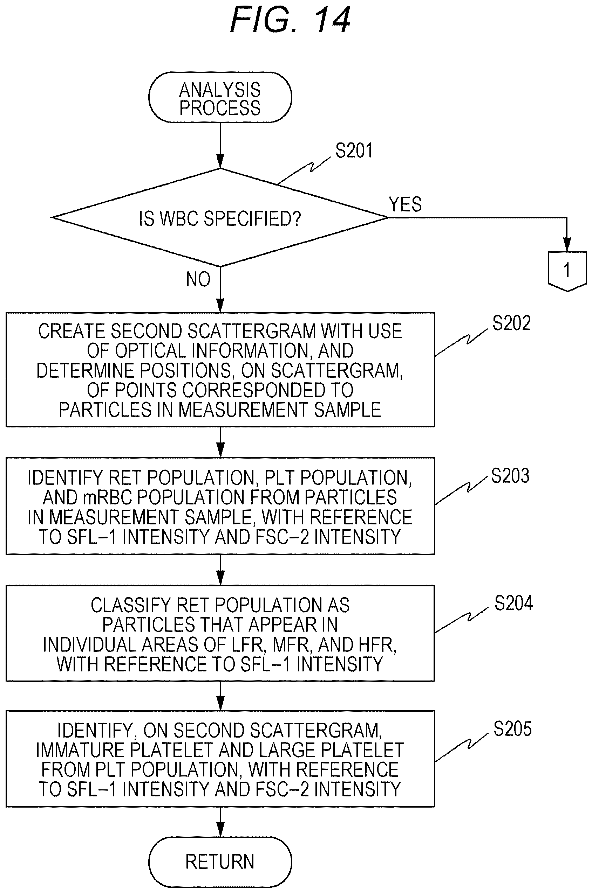

Exemplary analysis process in step S13 in Fig. 7 according to the second embodiment will be explained, while referring to Figs. 14 to 17, without limiting the invention. Platelet herein is also referred to as "PLT". Referring now to Fig. 14, upon acceptance by the controller 301 of the analysis unit 300 in step S201, of a designation of RET and PLT as the target to be measured, the process then advances to step S202. The measurement sample in this case is prepared without using the hemolysis reagent, and therefore contains mRBC and RET. The measurement sample also contains WBC and PLT. In this analysis process, the RET population and the PLT population are classified from the particles in the measurement sample. This identifies RET and PLT. Alternatively, the RET population, the PLT population, and the mRBC population may be classified from the particles in the measurement sample. This identifies RET, PLT, and RBC. This example will be described regarding an exemplary case where the RET population, the PLT population, and the RBC population are classified from the particles in a measurement sample, with reference to the fluorescence information and the third scattered light information. The fluorescence information is preferably the SFL-1 intensity. The third scattered light information is preferably the second forward-scattered light intensity (also referred to as "FSC-2 intensity").

The particles in the measurement sample may be classified, typically by analysis on the scattergrams. A scattergram for classifying the RET population and the PLT population from the particles in the measurement sample, with reference to the fluorescence information and the third scattered light information, is also referred to as "second scattergram". This example employs the SFL-1 intensity as the fluorescence information, and the FSC-2 intensity as the third scattered light information. The second scattergram is created typically with reference to the SFL-1 intensity and the FSC-2 intensity. Referring now to Fig. 14, the analysis unit 300 in step S202 creates the second scattergram. For example, the analysis unit 300 creates a scattergram having the SFL-1 intensity and the FSC-2 intensity plotted on two coordinate axes, with reference to the acquired optical information. Positions of points corresponded to the individual particles in the measurement sample are then determined, on the thus created second scattergram. The abscissa and the ordinate of the second scattergram may be expressed in either logarithmic or linear scale. The second scattergram, with the abscissa and the ordinate expressed in logarithmic scales, is suitable for PLT analysis. Meanwhile, the second scattergram, with the abscissa and the ordinate expressed in linear scales, is suitable for RET and RBC analyses.

In step S203, the analysis unit 300 classifies the blood cells other than WBC, into the RET population, the PLT population, and the mRBC population, with reference to the SFL-1 intensity and the FSC-2 intensity. The RET population demonstrates higher SFL-1 intensity, than the mRBC population. The PLT population demonstrates lower FSC-2 intensity, than the RET population. Now, the fluorescence intensity and the scattered light intensity of the particle populations on the scattergram may also be compared, typically with use of statistical representative values of the fluorescence intensity and scattered light intensity of the individual particle populations. The statistical representative values of the fluorescence intensity and the scattered light intensity of a certain particle population are values that can be acquired from the fluorescence intensity and the scattered light intensity of the particles that constitute the particle population. The statistical representative values are exemplified by median value, mean value, and mode value. The median value is preferred.

Fig. 15A illustrates examples of the second scattergram (in logarithmic scale) and the second scattergram (in linear scale). There are the RET population, the PLT population, and the mRBC population appeared on the individual second scattergrams. In the second scattergram (in logarithmic scale), the RET and mRBC populations appear in areas where the FSC-2 intensity is high, meanwhile the PLT population appears in an area where the FSC-2 intensity is low. In the second scattergram (in linear scale), the PLT population appears in an area where the FSC-2 intensity is low, meanwhile the RET population appears in an area where the FSC-2 intensity is higher than that of the PLT population. In both scattergrams, the RET population appears in the areas where the SFL-1 intensity is higher than that of the mRBC population. Paragraphs below will explain how the particles in the measurement sample are classified into the RET population, the mRBC population, and the PLT population, on the second scattergrams.

As has been described previously, the PLT has the smallest size among the blood cells, and thus produces the forward-scattered light whose intensity is lower than that of RET. The PLT population therefore appears on the second scattergram, in the area where the FSC-2 intensity is lower than that of the RET population. RET and PLT are thus discriminable, with reference to the FSC-2 intensity. As has been described previously, RET contains a small amount of ribonucleic acid, meanwhile mRBC contains neither nucleus nor nucleic acid. The RET population therefore appears in the area where the fluorescence intensity is higher than that of the mRBC population. mRBC and RET are thus discriminable, with reference to the SFL-1 intensity. As can be seen, the particles in the measurement sample are classified into the RET population, the PLT population, and the mRBC population, on the second scattergrams.

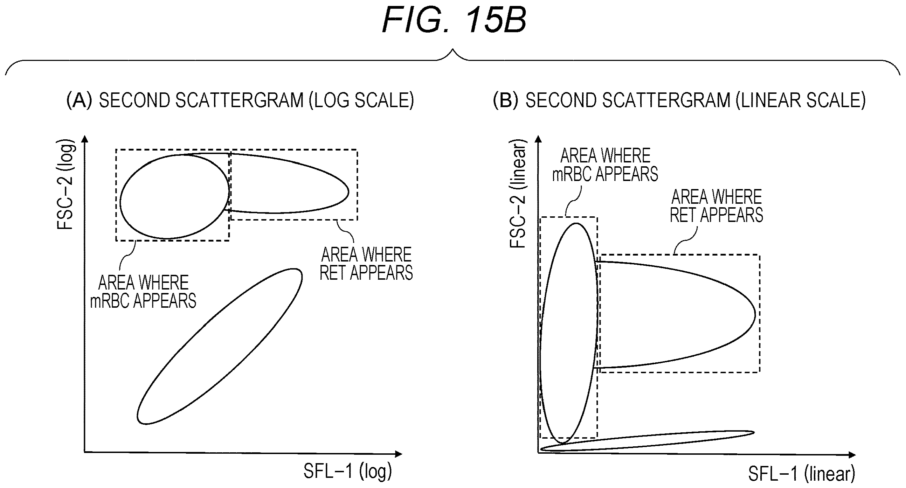

Referring now to Fig. 14, the analysis unit 300 in step S203 identifies the RET population, the PLT population, and the mRBC population from the particles in the measurement sample. RET matures to mRBC, and this makes it difficult to exactly define the boundary between the RET population and the mRBC population. It is therefore conceived, for example, to set a threshold value of the SFL-1 intensity at which the mRBC population and the RET population are dividable, typically with reference to a range of the SFL-1 intensity that can be usually demonstrated by mRBC, or an average level of the mRBC count. Such threshold value may be defined as a boundary between the mRBC population and the RET population on the second scattergram. The areas where mRBC and RET individually appear may be set with reference to the threshold value and the FSC-2 intensity, typically as indicated by broken lines in Fig. 15B. Particles in the thus set area where mRBC appears can be identified as mRBC. Meanwhile, particles in the thus set area where RET appears can be identified as RET. The population that appears in an area where the FSC-2 intensity is low may be identified by the user as the PLT population on the second scattergram (in logarithmic scale), if the population that appears in an area where the FSC-2 intensity is high, and the population that appears in an area where the FSC-2 intensity is lower are clearly separated. Alternatively, the areas where the RET, PLT, and mRBC populations will appear are preliminarily determined on the second scattergram, by accumulating measurement data from a plurality of specimens, and the particles that appears in the individual areas may be identified as RET, PLT and mRBC. The thus identified RET, PLT, and RBC may be optionally counted. The cells are counted with use of the analysis unit 300.

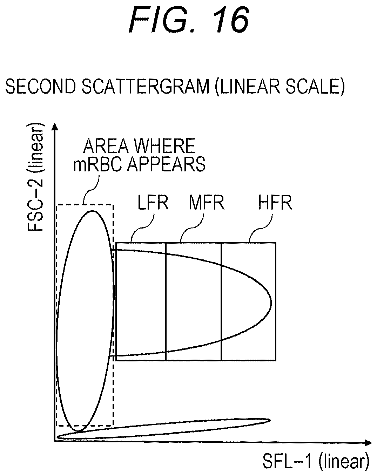

Referring now to Fig. 14, the process advances to step S204. In step S204, the analysis unit 300 classifies the RET population, into particle that appears in the LFR area, particle that appears in the MFR area, and particle that appears in the HFR area, with reference to the SFL-1 intensity. It has been known that the younger the RET, the more abundant the nucleic acid (RNA) in the cell. As RET matures, the RNA level in the cell decreases. That is, the RNA level in RET indicates the degree of maturation of RET. Now, the SFL-1 intensity of the cell measured by FCM reflects the nucleic acid level in the cell. The degree of maturation of RET may therefore be analyzed with reference to the SFL-1 intensity. Referring now to Fig. 16, the area where RET appears on the second scattergram (in linear scale) can be divided into three areas of LFR, MFR, and HFR with reference to the SFL-1 intensity. RET that appears in the individual areas may be counted. RET that appears in the HFR area is most nucleic acid-rich, and therefore represents most immature RET.

Alternatively, RET that appears in the individual areas of LFR, MFR, and HFR may be analyzed to acquire a mean value of the fluorescence intensity (SFL-1 intensity) and a mean value of the forward-scattered light intensity (FSC-2 intensity). Yet alternatively, the particle in an area where mRBC appears (that is, mRBC) may be analyzed to acquire mean values of the SFL-1 intensity and the FSC-2 intensity. Therapeutic effect on red blood cell diseases (for example, pernicious anemia, iron deficiency anemia, etc.) can be monitored with reference to the mean values of the SFL-1 intensity and FSC-2 intensity of RET and mRBC that appear in the individual areas, according to the method described in Japanese Examined Patent No. 4474135 (the entire content of which will be incorporated herein by reference). More specifically, the mean values of the SFL-1 intensity and the FSC-2 intensity in the individual areas of LFR, MFR, and HFR are plotted on a graph having the SFL-1 intensity and the FSC-2 intensity plotted on two coordinate axes, and an approximate straight line is acquired by the least squares method. A slope value of the approximate straight line is then acquired. The slope value is periodically acquired from the day the treatment starts, so as to monitor any change in the value, whereby the effect of the treatment can be determined.

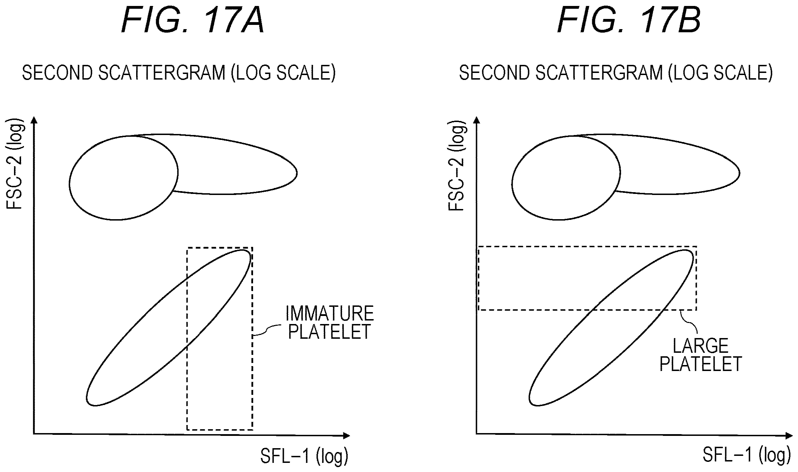

Referring now to Fig. 14, the process advances to step S205. In this step, the PLT population is further analyzed. In step S205, the analysis unit 300 identifies immature platelet and large platelet from the PLT population on the second scattergram (in logarithmic scale), with reference to the SFL-1 intensity and the FSC-2 intensity. As has been described previously, the mature platelet does not contain nucleic acid, meanwhile the immature platelet contains a small amount of ribonucleic acid. Hence, PLT that demonstrates high SFL-1 intensity may be identified as immature platelet, from among the PLT population on the second scattergram. For example, as illustrated in (A) in Fig. 17, PLT that appears in an area where SFL-1 is high (the area surrounded by the broken line) on the second scattergram, may be identified as immature platelet. PLT other than immature platelet may be identified as mature platelet. Thus, the PLT population is classified into immature platelet and mature platelet. The identified immature platelet may be counted. Immature platelet fraction (IPF) may be estimated, if the platelet count has already been acquired by counting the PLT population. IPF represents a ratio of platelet count in an area with high fluorescence intensity (the area surrounded by a solid-lined square), to the platelet count. IPF has been known as an index of platelet productivity.

Since the large platelet has larger size than that of normal PLT, so that PLT that demonstrates high FSC-2 intensity, from among the PLT population on the second scattergram, may be identified as the large platelet. For example, as illustrated in the second scattergram named (B) in Fig. 17, PLT that appears in an area where FSC-2 is high (the area surrounded by the broken line) may be identified as large platelet. PLT other than the large platelet may be identified as normal-sized PLT. Thus, the PLT population is classified into large platelet and normal-sized platelet. The identified large platelet may be counted. Percentage of large platelet (P-LCR) may be estimated, if the platelet count has already been acquired by counting the PLT population. P-LCR represents a ratio of platelet count in an area with high forward-scattered light intensity (the area surrounded by a broken-lined square), to the platelet count. P-LCR has been known as an index of platelet productivity and congenital diseases. After completion of the analysis of PLT, the analysis unit 300 finishes the analysis process. The process then advances to step S14 in Fig. 7.

Referring now to Fig. 14, upon acceptance, in step S201 by the controller 301 of the analysis unit 300, of a designation of WBC as the target to be measured, the process then advances to step S101 in Fig. 12. In this case, the analysis process (classification of WBC) of the first embodiment takes place. After completion of the classification of WBC, the analysis unit 300 finishes the analysis process. The process then advances to step S14 in Fig. 7.

Referring now to Fig. 7, upon completion of the aforementioned analysis process, the controller 301 in step S14 outputs analysis result on the display 305. The process thus finishes. The paragraphs above have described the process for analyzing WBC in the measurement sample, while referring to Figs. 12 and 13. The paragraphs above have also described the process for analyzing reticulocyte and platelet in the measurement sample, while referring to Figs. 14 to 17. The analysis process may, however, only be implemented in a part thereof, or may be implemented entirely.

An analysis result screen that is output on the display 305 may contain, for example, the number of subpopulations of white blood cell; reticulocyte count; platelet count; particle counts in the individual areas of LFR, MFR, and HFR; IPF; and P-LCR. Besides these information, the screen may contain a scattergram created with reference to the optical information. The screen may further contain a flag based on the analysis result.

As has been described, the method for analyzing blood cells of this embodiment can provide a medical worker such as doctor or medical technician, with analysis result of white blood cell, such as information on the number of subpopulations of white blood cell contained in the specimen. The method can also provide analysis result of reticulocyte and platelet, such as information on the counts of reticulocyte and platelet contained in the specimen. The method can yet also provide analysis result of red blood cell. The medical worker can make decision from the acquired analysis results, on whether the specimen should further be inspected or not. All of the aforementioned preparation of the measurement sample and analysis of the specimen take place in vitro.

The present invention will be further detailed below, while referring to Examples, to which the present invention is however not limited.

### EXAMPLES

### Example 1: Classification of White Blood Cell into Subpopulations

The white blood cell in the blood specimen was classified into the subpopulations by the method for analyzing blood cells of this embodiment, with use of various fluorescent dyes which can be excited by the light of the first wavelength (315 nm or longer and 600 nm or shorter). Also results of the classification of white blood cell were compared with those from the prior method.

### (1) Blood Specimens

Whole blood (peripheral blood) was collected from a plurality of subjects, with use of an EDTA-2K blood collection tube, and stored at room temperature (25 ± 2°C). The whole blood was fractionated from the blood collection tube, to be used as each blood specimen. From among the blood specimens, those determined to contain 2% or more of immature granulocyte and 2% or more of reticulocytes, by analysis with an automated hematology analyzer XR-20 (Sysmex Corporation), were used in Example 1.

### (2) Reagents

Lysercell (registered trademark) WDF II (Sysmex Corporation) was used as the hemolysis reagent. Each staining reagent was prepared by dissolving each of various fluorescent dyes in a special-grade ethylene glycol (1 L). The fluorescent dyes used herein were
3-ethyl-2-[(3-ethyl-2-benzothiazolinylidene)methyl]benzothiazolium, 1,3,3-trimethyl-2-[(3-methyl-2-benzoxazolinylidene)methyl]-3H-indolium, 3-ethyl-2-[(3-ethyl-2-benzothiazolinylidene)methyl]benzoxazolium, 3-ethyl-2-[(3-ethyl-6-methyl-2-benzothiazolinylidene)methyl]-6-methyl-benzothiazolium, and
3-ethyl-6-methyl-2-[(3-ethyl-6-methyl-2-benzothiazolinylidene)methyl]benzothiazolium. Fluorocell (registered trademark) WDF (Sysmex Corporation) was used for comparison. Fluorocell WDF is a staining reagent that contains a fluorescent dye excited by the light of the second wavelength (610 nm or longer and 750 nm or shorter). The measurement sample prepared with use of Fluorocell WDF and Lysercell WDF II is also referred to as "Comparative Example 1", hereinafter.

### (3) Analyzer

A prototype, modified from a flow cytometer XF-1600 (Sysmex Corporation) was used as the analyzer. The prototype was fabricated as follows. The original XF-1600 has been equipped with three semiconductor laser light sources that emit blue-violet (405 nm) light, blue light (488 nm) and red (639 nm) light, detectors for scattered light of the individual colors, and detectors for fluorescence excited by lights of the individual colors. The XF-1600 was then modified, by detaching the semiconductor laser light source that emits blue (488 nm) light, to an FCM system having two semiconductor laser light sources. The prototype has the hardware design illustrated in Figs. 1 to 3, and an FCM detector has a structure illustrated in Fig. 4. For the measurement of Comparative Example 1, an automated hematology analyzer XR-20 (Sysmex Corporation) was used. XR-20 has been equipped with a semiconductor laser light source that emits red (639 nm) light, a detector for red forward-scattered light, a detector for red side-scattered light, and a detector for fluorescence (660 nm or longer) excited by the red light.

### (4) Method of Measurement

The preparation and measurement of the measurement sample were implemented according to a manual attached to XF-1600, except that the aforementioned staining reagents were used. The measurement sample was prepared by mixing 1000 µL of Lysercell (registered trademark) WDF II, 17 µL of whole blood, and 0.5 to 2 µL of each staining reagent. The final concentration of each fluorescent dye in the measurement sample was 0.1 to 0.5 ppm. The measurement sample was measured after incubation at 41°C for 25 seconds. Data was analyzed with the aid of software installed on the analyzer. In Comparative Example 1, XR-20 (Sysmex Corporation) was used for the measurement, and XR-WDF channel was used for the analysis.

### (5) Analysis

The individual measurement samples were measured with the analyzer, to acquire the fluorescence information, the first scattered light information, and the second scattered light information. The fluorescence information corresponded to the fluorescence intensity, obtained by irradiating the particles in the measurement sample with a blue-violet laser (also referred to as "fluorescence intensity (blue-violet)" or "V-SFL", hereinafter). In Comparative Example 1, the fluorescence intensity, obtained by irradiating the particles in the measurement sample with a red laser (also referred to as "fluorescence intensity (red)" or "R-SFL", hereinafter), was acquired as the fluorescence information. The first scattered light information corresponded to the side-scattered light intensity, obtained by irradiating the particles in the measurement sample with a blue-violet laser (also referred to as "side-scattered light intensity (blue-violet)" or "V-SSC", hereinafter). The second scattered light information was the side-scattered light intensity obtained by irradiating the particles in the measurement sample with a red laser (also referred to as "side-scattered light intensity (red)" or "R-SSC", hereinafter).

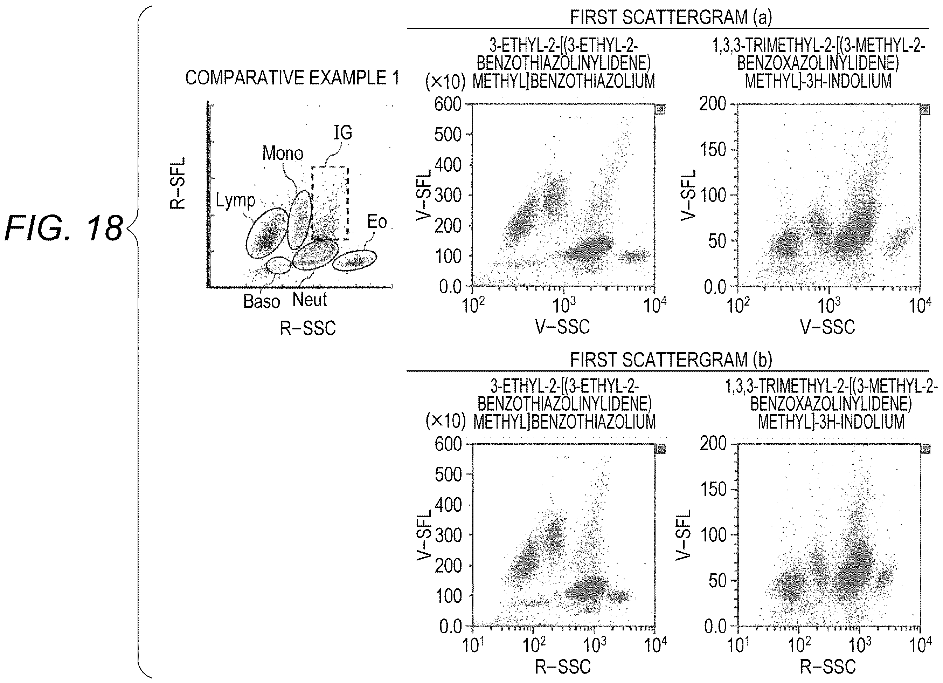

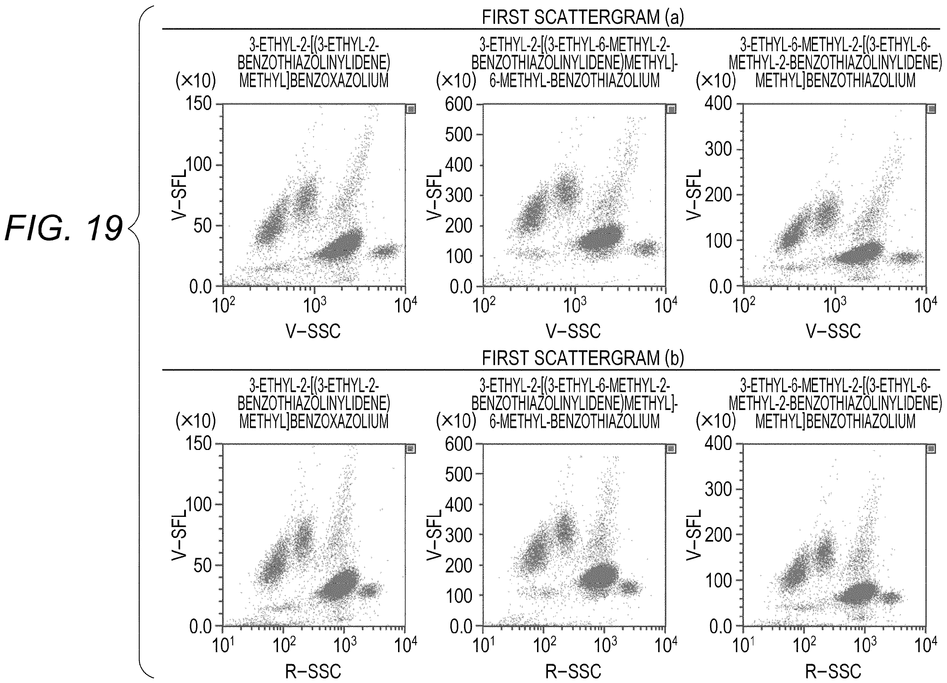

Scattergrams were created for the individual measurement samples, with the side-scattered light intensity (blue-violet) plotted on the abscissa, and with the fluorescence intensity (blue-violet) plotted on the ordinate (also referred to as "first scattergram (a)", hereinafter). Also scattergrams were created for the individual measurement samples, with the side-scattered light intensity (red) plotted on the abscissa, and with the fluorescence intensity (blue-violet) plotted on the ordinate (also referred to as "first scattergram (b)", hereinafter). In Comparative Example 1, a scattergram was created with the side-scattered light intensity (red) plotted on the abscissa, and with the fluorescence intensity (red) plotted on the ordinate. The individual scattergrams are illustrated in Figs. 18 and 19. Note that the analysis result of Comparative Example 1 corresponds to the classification result of white blood cell by a prior method. In Fig. 18, "Lymp" stands for lymphocyte, "Mono" for monocyte, "Neut" for neutrophil, "Eo" for eosinophil, "Baso" for basophil, and "IG" for immature granulocyte.

Referring now to Fig. 18, the scattergram of Comparative Example 1 demonstrated that white blood cell was classified into five subpopulations of lymphocyte, monocyte, neutrophil, eosinophil, and basophil. Also a population of immature granulocyte was observed in an area where the fluorescence intensity is higher than that of the neutrophil population. Referring now to Figs. 18 and 19, white blood cell was classified into five subpopulations on the first scattergrams (a) and (b), when analyzed with use of any of the aforementioned fluorescent dyes. In any of all scattergrams, the immature granulocyte population was observed in an area where the fluorescence intensity is higher than that of the neutrophil population, and the side-scattered light intensity is higher than that in the monocyte population. As can be seen, the method for analyzing blood cells of this embodiment was proved to classify white blood cell, similarly to the prior method. The method was also proved to count white blood cell and the individual subpopulations, by counting the particles contained in each of the thus classified population.

### Example 2: Classification of Reticulocyte and Platelet

Reticulocyte and platelet in the blood specimen were classified by the method for analyzing blood cells of this embodiment, with use of various fluorescent dyes which can be excited by the light of the first wavelength. Also results of the classification of reticulocyte and platelet were compared with those from the prior method.

### (1) Blood Specimen and Reagents

In Example 2, the same blood specimen as in Example 1 was used. CELLPACK (registered trademark) DFL (Sysmex Corporation) was used as the dilution reagent. The staining reagent was prepared in the same manner as in Example 1. The fluorescent dyes used herein were the same as in Example 1. Fluorocell (registered trademark) RET (Sysmex Corporation) and Fluorocell (registered trademark) PLT (Sysmex Corporation) were used for comparison. Fluorocell RET and Fluorocell PLT are staining reagents each containing a fluorescent dye excited by the light of the second wavelength (610 nm or longer and 750 nm or shorter). The measurement sample prepared with use of Fluorocell RET and CELLPACK DFL is also referred to as "Comparative Example 2", hereinafter. The measurement sample prepared with use of Fluorocell PLT and CELLPACK DFL is also referred to as "Comparative Example 3", hereinafter.

### (2) Analyzer and Method for Measurement

A prototype, modified from XF-1600 (Sysmex Corporation) was used as the analyzer, similarly to as in Example 1. Meanwhile, XR-20 (Sysmex Corporation) was used for the measurement in Comparative Examples 2 and 3. The preparation and measurement of the measurement sample were implemented according to a manual attached to XF-1600, except that CELLPACK DFL and the aforementioned staining reagents were used. The measurement sample was prepared by mixing 1000 µL of CELLPACK DFL, 17 µL of whole blood, and 20 µL of the staining reagent. The final concentration of each fluorescent dye in the measurement sample was 51.25 ppm. The measurement sample was measured after incubation at 41°C for 30 seconds.

### (3) Analysis

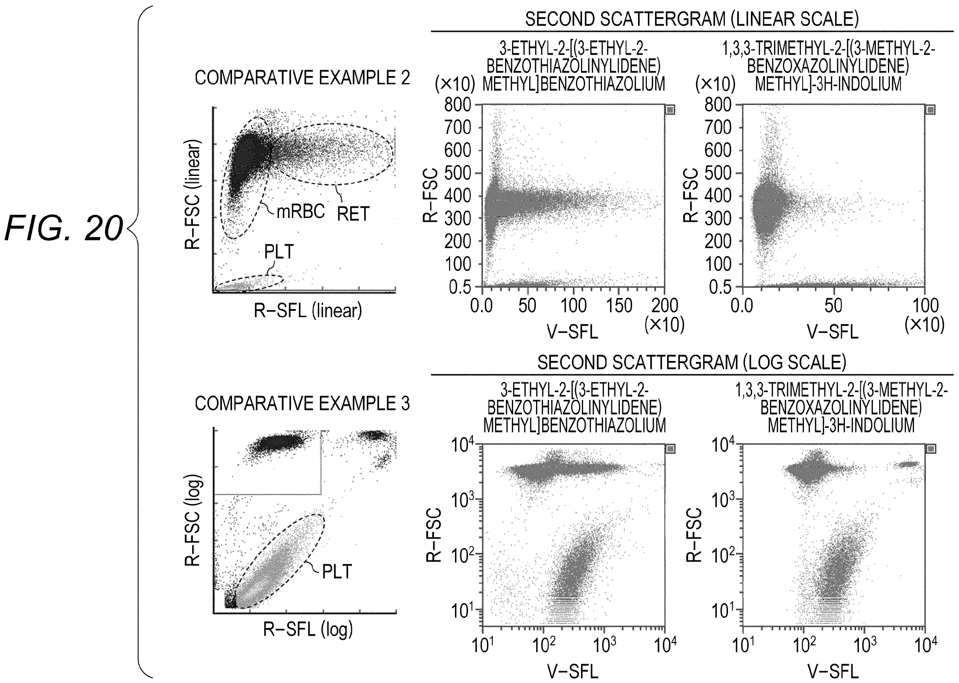

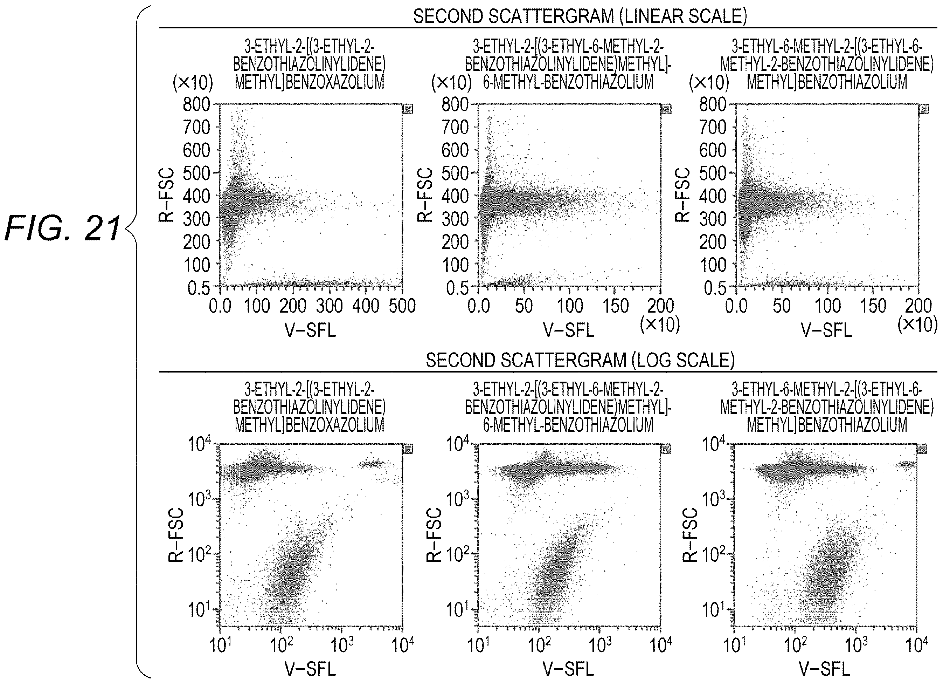

The individual measurement samples were measured with the analyzer, to acquire the fluorescence information, and the third scattered light information. The fluorescence information corresponds to the fluorescence intensity (blue-violet). The fluorescence intensity (red) was obtained, for Comparative Examples 2 and 3. The third scattered light information corresponds to the forward-scattered light intensity, obtained by irradiating the particles in the measurement sample with a red laser (also referred to as "forward-scattered light intensity (red)" or "R-FSC", hereinafter). The scattergrams were created for the individual measurement samples, with the fluorescence intensity (blue-violet) plotted on the abscissa, and the forward-scattered light intensity (red) plotted on the ordinate (also referred to as "second scattergram", hereinafter). The second scattergrams were created in two ways, in linear scale and in logarithmic scale. The individual scattergrams are illustrated in Figs. 20 and 21. Analysis result of Comparative Example 2 corresponds to the classification result of reticulocyte by the prior method, meanwhile analysis result of Comparative Example 3 corresponds to the classification result of platelet by the prior method.

Referring now to Fig. 20, the scattergram of Comparative Example 2 demonstrated the mature red blood cell population appeared in an area where R-SFL is low and R-FSC is high. The reticulocyte population appeared in an area where R-SFL is higher than that of the mature red blood cell population. The platelet population appeared in an area where R-FSC is low. As can be seen, mature red blood cell, reticulocyte, and platelet were clearly classified on the scattergram of Comparative Example 2. Referring now to Figs. 20 and 21, mature red blood cell, reticulocyte, and platelet were clearly classified on the second scattergrams in a linear scale, when analyzed with use of any of the aforementioned fluorescent dyes. Hence, the method for analyzing blood cells of this embodiment was proved to classify mature red blood cell and reticulocyte, similarly to the prior method.

Referring now to Fig. 20, the scattergram of Comparative Example 3 demonstrated the platelet population appeared in an area where R-FSC and R-SFL are low. As can be seen, platelet was clearly classified from the particles in the measurement sample, on the scattergram of Comparative Example 3. Referring now to Figs. 20 and 21, the populations of mature red blood cell and reticulocyte appeared in areas where R-FSC is high on the second scattergrams in a logarithmic scale, when analyzed with use of any of the aforementioned fluorescent dyes. The platelet population appeared in an area where R-FSC is lower than that of the populations of mature red blood cell and reticulocyte. The populations of mature red blood cell and the reticulocyte were clearly distinguishable from the platelet population. As can be seen, the method for analyzing blood cells of this embodiment was proved to classify platelet, similarly to the prior method.

## Claims

1. A method for analyzing blood cells, the method comprising:
irradiating a measurement sample that contains particles stained with a fluorescent dye with at least one light, to acquire fluorescence information and scattered light information generated from the particle; and
classifying blood cells from the particles in the measurement sample, with reference to the fluorescence information and the scattered light information,
the fluorescent dye being represented by formula (I) below: 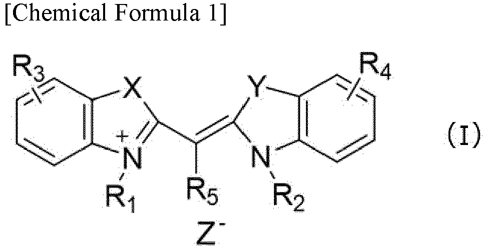 (where, each of R₁ and R₂, identically or differently, is a halogen, an alkyl group having 1 to 18 carbon atoms, an alkenyl group having 3 to 20 carbon atoms, an alkyl group having 1 to 18 carbon atoms and a hydroxy group, an alkyl group having 1 to 18 carbon atoms and a carboxy group, an alkyl group having 1 to 18 carbon atoms and a sulfo group, -(CH₂)ₘ-NR₆R₇, -(CH₂)ₙ-O-R₈, or a benzyl group optionally having a substituent;
each of R₃ and R₄, identically or differently, is a hydrogen atom, a halogen, an alkyl group having 1 to 18 carbon atoms, a phenyl group optionally having a substituent, an alkoxy group having 1 to 6 carbon atoms, or -(CH₂)ₘ-O-R₈;
R₅ is a hydrogen atom, a halogen, an alkyl group having 1 to 6 carbon atoms, an alkyl group having 1 to 6 carbon atoms and a hydroxy group, an alkyl group having 1 to 6 carbon atoms and a carboxy group, an alkyl group having 1 to 6 carbon atoms and a sulfo group, a phenyl group optionally having a substituent, a benzyl group optionally having a substituent, or -(CH₂)ₙ-O-R₆;
each of R₆ and R₇, identically or differently, is a hydrogen atom or an alkyl group having 1 to 18 carbon atoms;
R₈ is a hydrogen atom, an alkyl group having 1 to 18 carbon atoms, or a phenyl group optionally having a substituent;
m is an integer of 1 to 18, and n is an integer of 1 to 6;
each of X and Y, identically or differently, is a sulfur atom, an oxygen atom, a selenium atom, or CR₉R₁₀;
each of R₉ and R₁₀, identically or differently, is an alkyl group having 1 to 3 carbon atoms; and
Z⁻ is a counter ion).

2. The method according to claim 1, wherein in formula (I) above, with either X or Y given by an oxygen atom, the other is a sulfur atom, a selenium atom, or C(CH₃)₂.

3. The method according to claim 1 or 2, wherein the fluorescent dye is at least one selected from the group consisting of 3-ethyl-2-[(3-ethyl-2-benzothiazolinylidene)methyl] benzothiazolium, 3-ethyl-2-[(3-ethyl-6-methyl-2-benzothiazolinylidene)methyl]-6-methyl-benzothiazolium, 3-ethyl-2-[(3-ethyl-2-benzothiazolinylidene)methyl]benzoxazolium, 3-ethyl-6-methyl-2-[(3-ethyl-6-methyl-2-benzothiazolinylidene)methyl]benzothiazolium, 3-methyl-2-[(1,3,3-trimethyl-2-indolinylidene)methyl]benzothiazolium, and 1,3,3-trimethyl-2-[(3-methyl-2-benzoxazolinylidene)methyl]-3H-indolium.

4. The method according to any of claims 1 to 3, wherein the measurement sample is prepared by mixing the fluorescent dye, a hemolysis reagent, and whole blood, and
in the step of classifying, white blood cell in the measurement sample is classified into subpopulations.

5. The method according to claim 4, wherein the at least one light is a light of a first wavelength from 315 nm or longer and 600 nm or shorter,
the fluorescence information is information related to fluorescence generated from the fluorescent dye on the particles upon irradiation of the measurement sample with the light of the first wavelength, and
the scattered light information is information related to side-scattered light generated from the particles upon irradiation of the measurement sample with the light of the first wavelength.

6. The method according to claim 4, wherein the at least one light is a light of a first wavelength from 315 nm or longer and 600 nm or shorter, and a light of a second wavelength from 610 nm or longer and 750 nm or shorter,
the fluorescence information is information related to fluorescence generated from the fluorescent dye on the particles upon irradiation of the measurement sample with the light of the first wavelength, and
the scattered light information is information related to side-scattered light generated from the particles upon irradiation of the measurement sample with the light of the first wavelength,
and information related to side-scattered light generated from the particles upon irradiation of the measurement sample with the light of the second wavelength.

7. The method according to claim 5 or 6, wherein the fluorescence information is fluorescence intensity, and the scattered light information is side-scattered light intensity, and
in the step of classifying, white blood cell in the measurement sample is classified into subpopulations, with reference to the fluorescence intensity and the side-scattered light intensity.

8. The method according to claim 7, wherein a scattergram is created with reference to the fluorescence intensity and the side-scattered light intensity, and the white blood cell is classified into subpopulations.

9. The method according to claim 8, wherein the subpopulations comprise lymphocyte population, monocyte population, and granulocyte population.

10. The method according to claim 8, wherein the subpopulations comprise lymphocyte population, monocyte population, neutrophil population, eosinophil population, and basophil population.

11. The method according to any of claims 1 to 3, wherein the measurement sample is prepared by mixing the fluorescent dye, a dilution reagent, and whole blood, and
in the step of classifying, reticulocyte and platelet are classified from the particles in the measurement sample.

12. The method according to claim 11, wherein the measurement sample is free of a hemolysis reagent.

13. The method according to claim 11 or 12, wherein the at least one light is a light of a first wavelength from 315 nm or longer and 600 nm or shorter, and a light of a second wavelength from 610 nm or longer and 750 nm or shorter,
the fluorescence information is information related to fluorescence generated from the fluorescent dye on the particles upon irradiation of the measurement sample with the light of the first wavelength, and
the scattered light information is information related to forward-scattered light generated from the particles upon irradiation of the measurement sample with the light of the second wavelength.

14. The method according to claim 13, wherein the fluorescence information is fluorescence intensity, and the scattered light information is forward-scattered light intensity, and
in the step of classifying, the particles in the measurement sample are classified into reticulocyte, platelet, and mature red blood cell, with reference to the fluorescence intensity and the forward-scattered light intensity.

15. The method according to claim 14, wherein a scattergram is created with reference to the fluorescence intensity and the forward-scattered light intensity, and the particles in the measurement sample are classified into reticulocyte population, platelet population, and mature red blood cell population, on the scattergram.

16. The method according to any of claims 1 to 15, wherein the first wavelength is 315 nm or longer and 490 nm or shorter.

17. A reagent for blood cell analysis, comprising the fluorescent dye represented by formula (I) above, for use in the method according to any of claims 1 to 16.

18. A reagent kit for blood cell analysis, comprising a staining reagent that contains the fluorescent dye represented by formula (I) above, and a hemolysis reagent, and for use in the method according to any of claims 1 to 16..

19. A method for fluorescently staining blood cells, the method comprising contacting a fluorescent dye with the blood cells, the fluorescent dye being represented by formula (I) below: 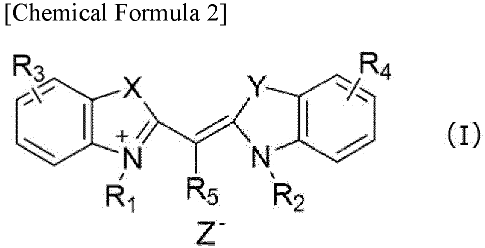 (where, each of R₁ and R₂, identically or differently, is a halogen, an alkyl group having 1 to 18 carbon atoms, an alkenyl group having 3 to 20 carbon atoms, an alkyl group having 1 to 18 carbon atoms and a hydroxy group, an alkyl group having 1 to 18 carbon atoms and a carboxy group, an alkyl group having 1 to 18 carbon atoms and a sulfo group, -(CH₂)ₘ-NR₆R₇, -(CH₂)ₙ-O-R₈, or a benzyl group optionally having a substituent;
each of R₃ and R₄, identically or differently, is a hydrogen atom, a halogen, an alkyl group having 1 to 18 carbon atoms, a phenyl group optionally having a substituent, an alkoxy group having 1 to 6 carbon atoms, or -(CH₂)ₘ-O-R₈;
R₅ is a hydrogen atom, a halogen, an alkyl group having 1 to 6 carbon atoms, an alkyl group having 1 to 6 carbon atoms and a hydroxy group, an alkyl group having 1 to 6 carbon atoms and a carboxy group, an alkyl group having 1 to 6 carbon atoms and a sulfo group, a phenyl group optionally having a substituent, a benzyl group optionally having a substituent, or -(CH₂)ₙ-O-R₆;
each of R₆ and R₇, identically or differently, is a hydrogen atom, or an alkyl group having 1 to 18 carbon atoms;
R₈ is a hydrogen atom, an alkyl group having 1 to 18 carbon atoms, or a phenyl group optionally having a substituent;
m is an integer of 1 to 18, and n is an integer of 1 to 6;
each of X and Y, identically or differently, is a sulfur atom, an oxygen atom, a selenium atom, or CR₉R₁₀;
each of R₉ and R₁₀, identically or differently, is an alkyl group having 1 to 3 carbon atoms; and
Z⁻ is a counter ion).
